# EUROPEAN PATENT APPLICATION

(11) **EP 4 134 652 A1**
(43) Date of publication of application: **15.02.2023**
(21) Application number: 21784194.9
(22) Date of filing: 08.04.2021
(51) Int. Cl.: G01N 1/10, A61B 10/02, A61B 90/50, A61G 12/00

(54) **ROBOT SYSTEM AND CONTROL METHOD FOR ROBOT SYSTEM**

(30) Priority: 10.04.2020 JP 2020071340; 29.05.2020 JP 2020094589
(71) Applicant: KAWASAKI JUKOGYO KABUSHIKI KAISHA, Kobe-shi, Hyogo 650-8670 (JP)
(72) Inventor: HASHIMOTO, Yasuhiko, Hyogo 650-8670 (JP); KAMEYAMA, Atsushi, Hyogo 650-8670 (JP); KAMON, Masayuki, Hyogo 650-8670 (JP); KOKUSHI, Hiroki, Hyogo 650-8670 (JP); RYU, Hideyuki, Hyogo 650-8670 (JP)
(74) Representative: Müller-Boré & Partner Patentanwälte PartG mbB
(86) International application number: PCT/JP2021/014930
(87) International publication number: WO 2021/206149

(57) **Abstract**

A robot system (100) includes a robot (101) includin g an arm (13) disposed in a first space (201), an operator (102) that receives an operation on a test instrument (50 ) in a second space (202), and a controller (110) that ope rates the test instrument (50) by controlling the robot (1 01) in response to the received operation.

## Description

### Technical Field

The present disclosure relates to a robot system and a control method for the robot system.

### Background Art

Conventionally, a self-propelled tray transfer robot that manages the presence or absence of a patient and is self-propelled to supply a blood collection tube containment tray of a patient, which is prepared in a blood collection tube preparation room, to a blood collection table is known (for example, see Patent Literature 1).

In the self-propelled tray transfer robot disclosed in Patent Literature 1, a non-contact medium such as an RF-ID is used as a test acceptance slip. A non-contact medium reader is installed at the entrance gate of the blood collection room to check the entry and exit of the patient.

Then, when the patient is absent, the self-propelled tray transfer robot receives the tray automatically prepared by a blood collection tube preparation device and stocks the received tray in a predetermined stock section. In addition, when the patient is present in a blood collection room, medical practice is assisted in a manner that a self-propelled tray transfer robot is self-propelled to supply a tray to the requested blood collection table.

### Citation List

### Patent Literature

[PTL 1] Japanese Unexamined Patent Publication No. 2007-130282

### Summary of Invention

In recent years, infectious diseases caused by coronaviruses such as SARS, MERS, and COVID-19 have spread. Infectious diseases caused by viruses other than coronavirus or various bacteria are also well known.

For patients suspected of having the infectious diseases, a medical staff collects a specimen such as a mucous membrane, performs a test such as a PCR test, and diagnoses the presence or absence of infection. On the other hand, there is a concern that the medical staff who collects the specimen will be infected with a virus or the like.

In addition, there is a concern that a medical staff who comes into contact with a patient infected with a virus or the like moves in the hospital during a period from when the test is performed until the infection is confirmed, so patients with other diseases in the hospital are infected with the virus or the like.

Therefore, the inventor of the present application has found that it is possible to sufficiently reduce the infection of a medical staff and the like in a hospital by causing a robot with a remote operation to perform a test and an examination on a patient suspected of being infected with a virus or the like.

The present disclosure has been made to solve the above-described problems, and one object of the present disclosure is to provide a robot system and a control method for the robot system capable of sufficiently reducing the infection of a medical staff and the like in a hospital.

According to a first aspect of the present disclosure, a robot system includes a robot that is disposed in a first space and includes an arm with a hand that holds a medical test instrument, an operator that receives an operation on the test instrument in a second space different from the first space, and a controller that operates the test instrument by controlling the robot in response to the received operation. The term "test instrument" has a concept that includes an instrument for examining a patient in addition to an instrument for testing a patient.

As a result, a medical staff can operate the robot with the operator in the second space isolated from a patient, so that it is possible to suppress an occurrence of a situation in which the medical staff and the like come into contact with the patient suspected of being infected with a virus or the like. Therefore, it is possible to sufficiently suppress the infection of a medical staff and the like in a hospital.

Furthermore, by separately using a robot for a patient suspected of having an infectious disease and a robot for a patient with another disease, it is possible to sufficiently suppress the infection to a patient with another disease.

According to a second aspect of the present disclosure, a control method for a robot system is a robot system control method including an arm that is disposed in a first space and includes a hand that holds a medical test instrument, and includes a step of receiving an operation on the test instrument in a second space different from the first space, and a step of operating the test instrument by controlling a robot in response to the received operation.

As a result, a medical staff can operate the robot with the operator in the second space isolated from a patient, so that it is possible to suppress an occurrence of a situation in which the medical staff and the like come into contact with the patient suspected of being infected with a virus or the like. Therefore, it is possible to provide a control method for a robot system capable of sufficiently suppressing infection of a medical staff or the like in a hospital.

Furthermore, it is possible to provide a control method for a robot system capable of sufficiently suppressing infection to a patient with another disease by separately using a robot for a patient suspected of having an infectious disease and a robot for the patient with another disease.

According to a third aspect of the present disclosure, a robot system includes a robot that is disposed in a first space and includes an arm with a hand that holds a medical test instrument, an imager that is provided at the hand or the arm and picks up an image of at least one of a nasal cavity and an oral cavity, an operator that receives an operation on the test instrument in a second space different from the first space, a controller that operates the test instrument by controlling the robot in response to the received operation, and a display disposed in the second space. The controller displays a pickup image picked up by the image pickup device on the display.

As a result, a medical staff can operate the robot with the operator in the second space isolated from a patient, so that it is possible to suppress an occurrence of a situation in which the medical staff and the like come into contact with the patient suspected of being infected with a virus or the like. Therefore, it is possible to provide a robot system capable of sufficiently suppressing infection of a medical staff or the like in a hospital.

Furthermore, it is possible to provide a robot system capable of sufficiently suppressing infection to a patient with another disease by separately using a robot for a patient suspected of having an infectious disease and a robot for the patient with another disease.

### Brief Description of Drawings

FIG. 1 is a schematic diagram illustrating a schematic configuration of a robot system according to a first embodiment of the present disclosure.
FIG. 2 is a schematic diagram illustrating the schematic configuration of the robot system according to the first embodiment of the present disclosure.
FIG. 3 is a schematic diagram illustrating a schematic configuration of a robot in the robot system illustrated in FIGS. 1 and 2.
FIG. 4 is a schematic diagram illustrating a schematic configuration of a hand of the robot in the robot system according to the first embodiment of the present disclosure.
FIG. 5 is a flowchart illustrating an example of an operation of the robot system according to the first embodiment of the present disclosure.
FIG. 6 is a schematic diagram illustrating an example of an operation of the robot in the robot system according to the first embodiment of the present disclosure.
FIG. 7 is a schematic diagram illustrating an example of image information and/or video information displayed on a first display illustrated in FIG. 1.
FIG. 8 is a schematic diagram illustrating another example of the image information and/or the video information displayed on the first display illustrated in FIG. 1.
FIG. 9 is a schematic diagram illustrating still another example of the image information and/or the video information displayed on the first display illustrated in FIG. 1.
FIG. 10 is a schematic diagram illustrating still yet another example of the image information and/or the video information displayed on the first display illustrated in FIG. 1.
FIG. 11 is a schematic diagram illustrating a schematic configuration of a robot system according to a first modification example in the first embodiment of the present disclosure.
FIG. 12 is a schematic diagram illustrating a schematic configuration of a robot system according to a second embodiment of the present disclosure.
FIG. 13 is a schematic diagram illustrating a schematic configuration of a robot system according to a third embodiment of the present disclosure.
FIG. 14 is a schematic diagram illustrating a schematic configuration of a robot system according to a fourth embodiment of the present disclosure.
FIG. 15 is a flowchart illustrating an example of an operation of the robot system according to the fourth embodiment of the present disclosure.
FIG. 16 is a schematic diagram illustrating a schematic configuration of a robot system according to a first modification example in the fourth embodiment of the present disclosure.
FIG. 17 is a schematic diagram illustrating a schematic configuration of a robot system according to a fifth embodiment of the present disclosure.
FIG. 18 is a flowchart illustrating an example of an operation of the robot system according to the fifth embodiment of the present disclosure.
FIG. 19 is a schematic diagram illustrating a schematic configuration of a robot system according to a sixth embodiment of the present disclosure.
FIG. 20 is a schematic diagram illustrating a schematic configuration of the robot system according to the sixth embodiment of the present disclosure.
FIG. 21 is a schematic diagram illustrating a schematic configuration of a hand of a robot illustrated in FIG. 19.
FIG. 22 is a flowchart illustrating an example of an operation of the robot system according to the sixth embodiment of the present disclosure.
FIG. 23 is a schematic diagram illustrating a schematic configuration of a robot system according to a first modification example in the sixth embodiment of the present disclosure.
FIG. 24 is a schematic diagram illustrating a schematic configuration of the robot system according to a seventh embodiment of the present disclosure.
FIG. 25 is a schematic diagram illustrating a schematic configuration of the robot system according to an eighth embodiment of the present disclosure.
FIG. 26 is a flowchart illustrating an example of an operation of the robot system according to the eighth embodiment of the present disclosure.
FIG. 27 is a block diagram illustrating a robot system according to a ninth embodiment of the present disclosure.
FIG. 28 is a diagram illustrating a robot in the robot system according to the ninth embodiment of the present disclosure.
FIG. 29 is a diagram illustrating an operator in the robot system according to the ninth embodiment of the present disclosure.
FIG. 30 is a diagram illustrating the robot and a patient according to the ninth embodiment of the present disclosure.
FIG. 31 is a diagram illustrating a cross section of a hand and a patient according to the ninth embodiment of the present disclosure.
FIG. 32 is a diagram illustrating an image in which a force and an insertion depth are displayed, according to the ninth embodiment of the present disclosure.
FIG. 33 is a diagram illustrating a profile of a patient according to the ninth embodiment of the present disclosure.
FIG. 34 is a diagram illustrating the profile and a model image of the patient according to the ninth embodiment of the present disclosure.
FIG. 35 is a flowchart illustrating a control method for the robot system according to the ninth embodiment of the present disclosure.
FIG. 36 is a diagram (1) illustrating an image in which a force and an insertion depth are displayed, according to a modification example.
FIG. 37 is a diagram (2) illustrating an image in which a force and an insertion depth are displayed, according to another modification example.

### Description of Embodiments

Hereinafter, embodiments of the present disclosure will be described with reference to the drawings. In all drawings, the same or corresponding portions are designated by the same reference signs, and duplicate description will be omitted. Further, in all the drawings, the components for describing the present disclosure are excerpted and illustrated, and the illustrations of other components may be omitted. Furthermore, the present disclosure is not limited to the following embodiments.

### (First Embodiment)

A robot system according to a first embodiment includes a robot, an operating machine, and a controller. The robot includes an arm which a hand that holds a medical test instrument and/or a medical examination instrument. The operating machine operates the robot. A first space in which the robot is disposed is isolated from a second space in which the operating machine is disposed. The controller (A) causes the robot to be self-propelled to approach a patient and (B) operates the arm based on operation command information of the arm and/or a hand, which is input from the operating machine, after (A) is performed.

Further, the robot system according to the first embodiment may further include a first imager and a first display that displays image information and/or video information obtained by picking-up of the first imager. The controller may be configured to perform (B) in a state where the first display displays the image information and/or the video information obtained by picking-up of the first imager.

Further, in the robot system according to the first embodiment, the robot and the operating machine may be configured by a master-slave method.

Further, in the robot system according to the first embodiment, a pair of laser light indicators may be disposed at the hand so that the rays of light with which irradiation is performed from the laser light indicators intersect with each other.

Further, in the robot system according to the first embodiment, the first imager may be disposed at the robot (a portion other than the hand, for example, an arm or the like), or may be disposed at the hand of the robot.

Further, in the robot system according to the first embodiment, the first display may be configured to display a virtual model showing position information of the medical test instrument and/or the medical examination instrument.

Further, in the robot system according to the first embodiment, an operation switch for an instruction to release holding the medical test instrument and/or the medical examination instrument may be disposed in the operating machine. The "operating machine" is an example of an "operator" in the claims.

In a control method for a robot system according to the first embodiment, the robot system includes a robot including an arm with a hand that holds a medical test instrument and/or a medical examination instrument, and an operating machine that operates the robot, and a first space in which the robot is disposed is isolated from a second space in which the operating machine is disposed. The control method includes (A) automatically moving the robot to the vicinity of a patient based on position information of the patient input from the operating machine and (B) operating the arm and/or the hand based on operation command information of the arm and/or the hand, which is input from the operating machine, after (A) is performed.

Further, in the control method for the robot system according to the first embodiment, the robot system may further include a first imager and a first display that displays image information and/or video information obtained by picking-up of the first imager. (B) may be performed in a state where the first display displays the image information and/or the video information obtained by picking-up of the first imager.

Further, in the control method for the robot system according to the first embodiment, the robot and the operating machine may be configured by a master-slave method.

Further, in the control method for the robot system according to the first embodiment, a pair of laser light indicators may be disposed at the hand so that the rays of light with which irradiation is performed from the laser light indicators intersect with each other.

Further, in the control method for the robot system according to the first embodiment, the first imager may be disposed at the robot (a portion other than the hand, for example, an arm or the like), or may be disposed at the hand of the robot.

Further, in the control method for the robot system according to the first embodiment, the first display may be configured to display a virtual model showing position information of the medical test instrument and/or the medical examination instrument.

Further, in the control method for the robot system according to the first embodiment, the first display may be configured to display a virtual model of a medical practice target site of a patient.

Further, in the control method for the robot system according to the first embodiment, an operation switch for an instruction to release holding the medical test instrument and/or the medical examination instrument may be disposed at the operating machine.

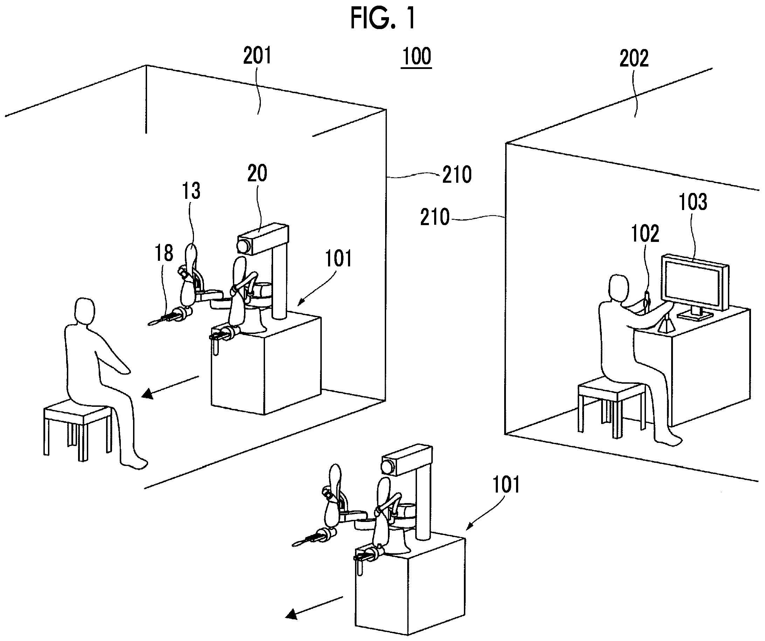

An example of the robot system according to the first embodiment will be described below with reference to FIGS. 1 to 10.

### [Configuration of Robot System]

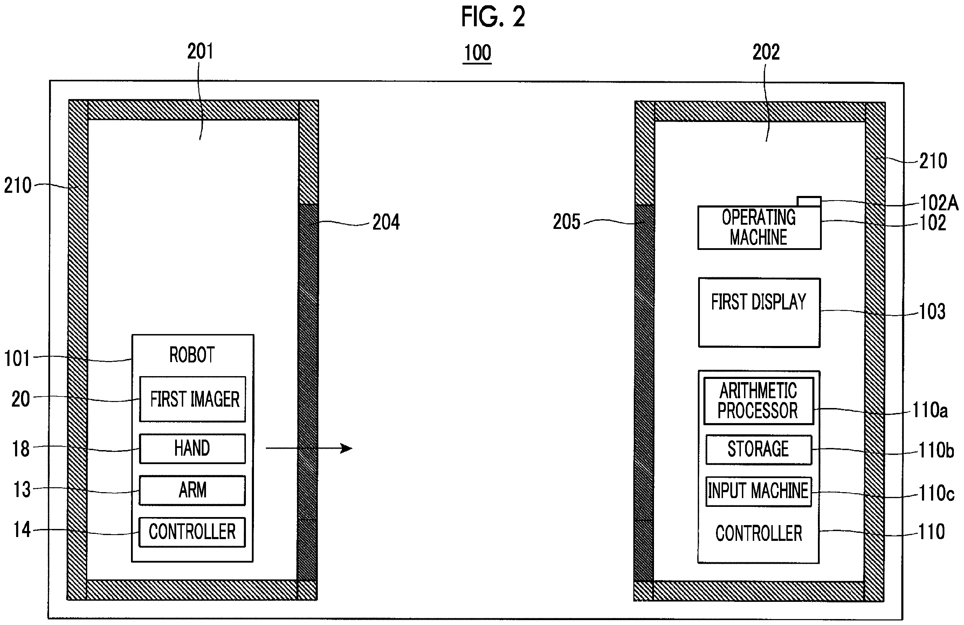

FIGS. 1 and 2 are schematic diagrams illustrating a schematic configuration of the robot system according to the first embodiment.

As illustrated in FIG. 1, a robot system 100 according to the first embodiment includes a robot 101, an operating machine 102, a first display 103, and a controller 110. The robot 101 is disposed in a first space 201. The operating machine 102, the first display 103, and the controller 110 are disposed in a second space 202.

The first space 201 and the second space 202 are different spaces separated from each other. The first space 201 and the second space 202 are isolated by a partition wall member 210.

An image pickup device (first imager) for picking up an image of a profile of a patient may be disposed in the first space 201. The image pickup device may be installed on the partition wall member 210 forming the first space 201, or may be gripped by a robot different from the robot 101.

Further, an instrument for performing a medical test by the robot 101, an experimental device, various test reagents, or the like may be disposed in a room (examination room/test room) constituting the first space 201. The instrument is, for example, an autopipette, a tip used for the autopipette, a microtube, a centrifuge tube, or a centrifuge settling tube. The experimental device is, for example, a centrifuge, a PCR device, or the like.

An anterior chamber may be provided in a room (operation room) constituting the second space 202. Further, a fan filter unit that makes the anterior chamber have negative pressure and makes the second space 202 (internal space of the operation room) have positive pressure may be installed in the anterior chamber. A known fan filter unit can be used as the fan filter unit.

Further, the partition wall member 210 may include a shutter (door) 204 that permits/prohibits movement into the first space 201, and may further include a shutter (door) 205 that permits/prohibits movement into the second space 202.

Further, the partition wall member 210 may be configured such that an operator (medical staff) or the like can view the inside of the first space 201 from the outside by forming a portion of the partition wall member 210 with a transparent member such as a glass plate.

The operating machine 102 operates the robot 101. As the operating machine 102, for example, a known operating machine such as a joystick, a keyboard, a numeric keypad, or a teaching pendant can be used.

Further, the operating machine 102 may be provided with a device that transmits force sense information detected by a force sense sensor (described later) provided at a hand 18 of the robot 101 and audio information to an operator. Such a device is, for example, a vibration motor, a speaker, a mechanism for expanding and contracting a housing constituting a grip portion, and the like.

The operating machine 102 may be configured to be portable so that the operator (medical staff) can carry the operating machine 102. Further, the robot 101 and the operating machine 102 may be configured by a master-slave method.

Further, the operating machine 102 may be provided with a release button 102A for releasing a medical test instrument or a medical examination instrument held by the hand 18 (releasing holding) in an emergency (for example, when the robot 101 performs an abnormal operation). When the release button 102A is pressed by the operator, the controller 110 may operate the robot 101 to separate the hand 18 from the patient.

The first display 103 displays image information and/or video information obtained by picking-up of the first imager 20 described later. The first display 103 may be configured by, for example, a stationary type display that is used by being stationary on a desk, floor, or the like. Further, the first display 103 may include a head-mounted display or glasses worn and used by the operator.

The robot 101 can be self-propelled to the vicinity of the patient based on the position information of the patient input from the operating machine 102 and/or position information in a hospital (for example, position information of a hospital room and an examination room).

Further, the robot 101 operates the arm and/or the hand based on operation information of the arm and/or the hand, which is input from the operating machine 102. At this time, the robot 101 may be configured so that the robot 101 automatically moves to the patient under control of the controller 110 in accordance with the work content of medical practice (for example, examination and/or test) to be performed such that a distance between the robot 101 and the patient is maintained to a predetermined first distance set in advance.

For example, in a case where the work (examination) of auscultating the front of the patient with a stethoscope is performed and then the work (examination) of auscultating the back of the patient with a stethoscope is performed, when the patient directs the back of the patient toward the robot 101, the robot 101 may automatically move backward and then automatically move to reduce a distance from the patient (maintaining the first distance).

Thus, the medical staff can operate the robot 101 by a remote operation and perform medical practice on the patient.

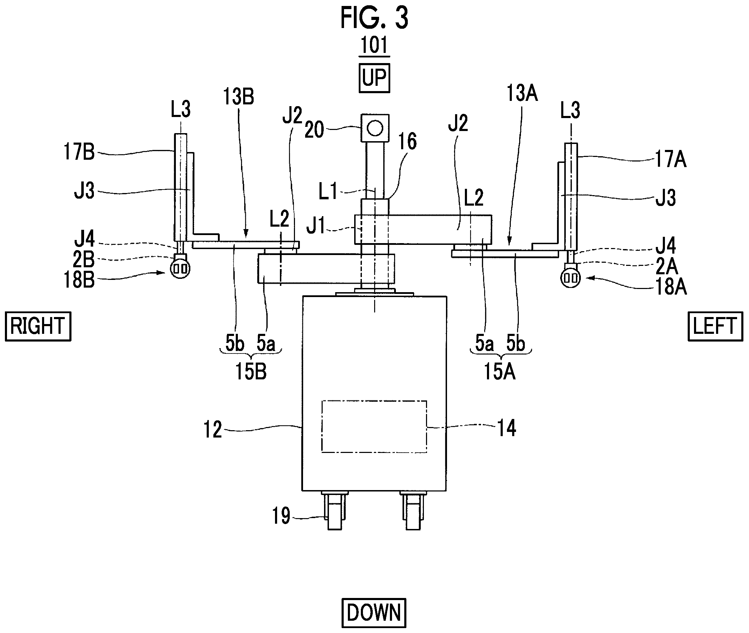

Here, the configuration of the robot 101 will be described in detail with reference to FIG. 3. A horizontal articulated dual-arm robot will be described below as the robot 101. Another robot such as a horizontal articulated robot or a vertical articulated robot may be adopted as the robot 101.

FIG. 3 is a schematic diagram illustrating a schematic configuration of the robot in the robot system illustrated in FIGS. 1 and 2. In FIG. 3, an up-down direction in the robot is represented as an up-down direction in FIG. 3.

As illustrated in FIG. 3, the robot 101 includes a carriage 12, a first arm 13A, a second arm 13B, a first hand 18A, a second hand 18B, and a controller 14 disposed in the carriage 12. The first arm 13A and The second arm 13B are mounted on the carriage 12.

When the first arm 13A and the second arm 13B are not distinguished from each other, the first arm 13A and the second arm 13B are simply referred to as the arm 13. Similarly, when the first hand 18A and the second hand 18B are not distinguished from each other, the first hand 18A and the second hand 18B are simply referred to as the hand 18.

Further, in the first embodiment, a form in which the controller 14 is disposed in the carriage 12 has been adopted. The present disclosure is not limited to this, and the controller 14 may be disposed outside the carriage 12. The controller 14 will be described later.

Wheels 19 are disposed on the lower surface of the carriage 12. An appropriate gear and a drive motor are connected to the wheels 19. Thus, the robot 101 can be self-propelled.

Further, a base shaft 16 and a first imager 20 are fixed to the upper surface of the carriage 12. The first imager 20 picks up an image and/or a video, and outputs the image information and/or video information obtained by pickup to the controller 110. The first imager 20 may be, for example, a video camera or an X-ray image pickup device.

The first imager 20 may be configured to output the image information and/or video information obtained by pickup to the first display 103 without passing through the controller 110. Further, the first imager 20 may be gripped by an arm other than the first arm 13A and the second arm 13B.

The base shaft 16 is provided with the first arm 13A and the second arm 13B to be rotatable around a rotation axis L1 passing through the axis of the base shaft 16. Specifically, the first arm 13A and the second arm 13B are provided to have a vertical height difference. The first arm 13A and the second arm 13B can operate independently or in relation to each other.

The first arm 13A has a first arm portion 15A, a first wrist portion 17A, a first hand 18A, and a first mounting portion 2A. Similarly, the second arm 13B has a second arm portion 15B, a second wrist portion 17B, a second hand 18B, and a second mounting portion 2B. Since the second arm 13B is configured in the similar manner to the first arm 13A, detailed description thereof will be omitted.

In the first embodiment, the first arm portion 15A is configured by a first link 5a and a second link 5b having a substantially rectangular parallelepiped shape. In the first link 5a, a rotating joint J1 is provided at the proximal end portion and a rotating joint J2 is provided at the distal end portion. Further, in the second link 5b, a linear motion joint J3 is provided at the distal end portion.

The proximal end portion of the first link 5a is joined to the base shaft 16 via the rotating joint J1, and the rotating joint J1 allows the first link 5a to rotate around the rotation axis L1. Further, the proximal end portion of the second link 5b is joined to the distal end portion of the first link 5a via the rotating joint J2, and the second link 5b can rotate around a rotation axis L2 by the rotating joint J2.

The first wrist portion 17A is joined to the distal end portion of the second link 5b via the linear motion joint J3 so as to be movable up and down with respect to the second link 5b. A rotating joint J4 is provided at the lower end portion of the first wrist portion 17A, and the first mounting portion 2A is provided at the lower end portion of the rotating joint J4.

The first mounting portion 2A is configured to be able to detachably attach the first hand 18A. Specifically, for example, the first mounting portion 2A includes a pair of rod members between which a distance is adjustable. The first hand 18A is sandwiched between the pair of rod members, and thus the first hand 18A can be attached to the first wrist portion 17A. As a result, the first hand 18A can rotate around a rotation axis L3 by the rotating joint J4. The distal end portion of the rod member may be bent.

The first hand 18A may be in any form as long as the first hand 18A is configured to hold a medical test instrument or a medical examination instrument. For example, as illustrated in FIGS. 1 and 3, the first hand 18A may be configured to hold a medical test instrument or a medical examination instrument by two claws. The medical test instrument may be, for example, a sterilized cotton swab, various tubes such as a tube with a screw cap, a syringe, a catheter, or an endoscopy test instrument. Further, the medical examination instrument may be, for example, a stethoscope or a tongue depressor.

In addition, the first hand 18A holds various workpieces such as drugs, meals, and test reagents, and can release (release the holding) the workpieces.

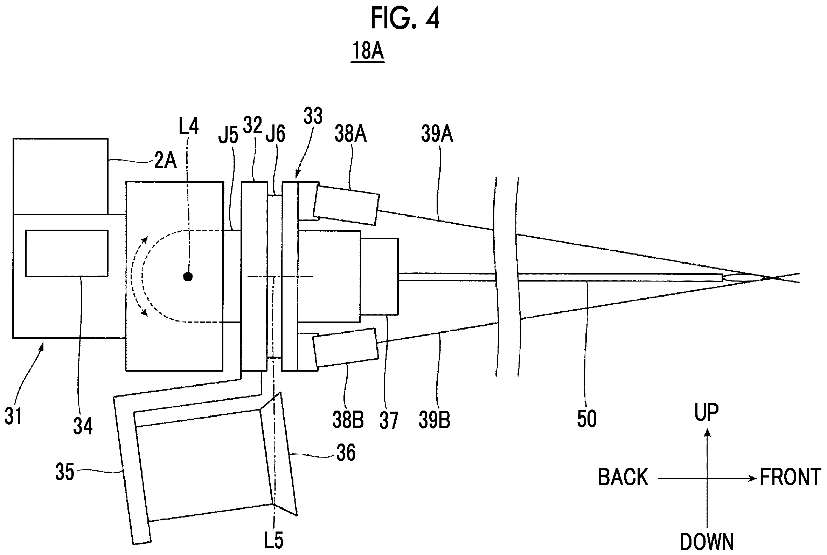

Here, another example of the first hand 18A (hand 18) will be described with reference to FIG. 4.

FIG. 4 is a schematic diagram illustrating a schematic configuration of the hand of the robot in the robot system according to the first embodiment. In FIG. 4, an up-down direction and a front-back direction in the robot are represented as an up-down direction and a front-back direction in FIG. 4.

As illustrated in FIG. 4, the first hand 18A includes a main body 31, an intermediate member 32, and a holding member 33. The main body 31 and the intermediate member 32 are joined to each other via a rotating joint J5. Further, the intermediate member 32 and the holding member 33 are joined to each other via a rotating joint J6. As a result, the holding member 33 can rotate around a rotation axis L4 and/or a rotation axis L5 with respect to the main body 31.

The main body 31 is provided with an actuator 34 for rotating the holding member 33. The actuator 34 may be, for example, a servomotor servo-controlled by the controller 14. Further, the main body 31 is provided with a rotation sensor (not illustrated) that detects the rotation position of the servomotor and a current sensor (not illustrated) that detects a current for controlling the rotation of the servomotor. The rotation sensor may be, for example, an encoder. Position information detected by the rotation sensor and current information detected by the current sensor may be output to the controller 110 via the controller 14.

A support member 35 is provided at the lower end portion of the intermediate member 32. A camera (first imager) 36 is attached to the support member 35. The camera 36 picks up an image and/or a video, and outputs the image information and/or video information obtained by pickup to the controller 110. The camera 36 may be, for example, a video camera or an X-ray image pickup device.

Further, a target picked up by the camera 36 may be the nostril of the patient, for example, when the nasopharyngeal swab is collected with a sterilized cotton swab. Further, the target picked up by the camera 36 may be, for example, the oral cavity of the patient when saliva or a specimen derived from the lower respiratory tract (sputum or the like) is collected by a suction catheter or the like.

In the first embodiment, a form in which the support member 35 and the camera 36 are disposed at the lower end portion of the intermediate member 32 has been adopted, but the present disclosure is not limited to this. The support member 35 and the camera 36 may be disposed at the upper end portion of the intermediate member 32 or the like. Further, the support member 35 and the camera 36 may be disposed at the holding member 33.

A chuck mechanism 37 for holding/releasing (releasing the holding) a medical test instrument or a medical examination instrument is attached to the holding member 33. The chuck mechanism 37 may be configured by, for example, an air chuck. Here, the chuck mechanism 37 holds a sterilized cotton swab 50 for collecting a specimen for a PCR test.

Further, a pair of laser pointers (laser light indicators) 38A and 38B are disposed at the holding member 33. The laser pointers 38A and 38B are disposed so that rays of laser light 39A and 39B checked from the respective laser pointers 38A and 38B intersect with each other in front of the first hand 18A. In addition, three or more laser light indicators may be disposed at the first hand 18A.

As a result, when the first hand 18A approaches the patient, a distance between the laser light 39A and the laser light 39B that hit the patient becomes smaller. Further, when the first hand 18A approaches the patient, the laser light that hits the patient becomes one point. Further, when the first hand 18A approaches the patient, the distance between the laser light 39A and the laser light 39B that hit the patient increases.

Therefore, the operator (medical staff) can easily understand a distance between the patient, and the distal end portion of the medical test instrument and/or the medical examination instrument (sterilized cotton swab 50) by the rays of laser light 39A and 39B with which irradiation is performed from the pair of laser pointers 38A and 38B.

Further, each of the joints J1 to J4 of the first arm 13A and the second arm 13B is provided with a drive motor as an example of an actuator that relatively rotates, or raises and lowers two members joined to each other by each joint (not illustrated). The drive motor may be, for example, a servomotor servo-controlled by the controller 14. Further, each of the joints J1 to J4 is provided with a rotation sensor (not illustrated) that detects the rotation position of the drive motor, and a current sensor (not illustrated) that detects the current for controlling the rotation of the drive motor. The rotation sensor may be, for example, an encoder. Position information detected by the rotation sensor and current information detected by the current sensor may be output to the controller 110 via the controller 14.

The controller 14 includes an arithmetic processor and a storage (not illustrated). The arithmetic processor is configured by a microprocessor, a CPU, and the like, and controls various operations of the robot 101 by reading and executing software such as a basic program stored in the storage.

The storage stores types of information such as basic programs and various types of fixed data. For example, map information in the hospital may be stored in the storage in advance.

The storage does not have to be single and may be configured as a plurality of storages (for example, random access memory and hard disk drive). When the arithmetic processor is configured by a microcomputer, at least a portion of the storage may be configured as an internal memory of the microcomputer or may be configured as an independent memory.

Further, the controller 14 may control various operations of the robot 101 based on various types of command information input from the controller 110.

As illustrated in FIGS. 1 and 2, the controller 110 includes an arithmetic processor 110a, a storage 110b, and an input machine (operating machine) 110c. The arithmetic processor 110a is configured by a microprocessor, a CPU, and the like, and controls various operations of the robot system 100 by reading and executing software such as a basic program stored in the storage 110b.

The storage 110b stores types of information such as basic programs and various types of fixed data. The storage 110b does not have to be single and may be configured as a plurality of storages (for example, random access memory and hard disk drive). When the arithmetic processor 110a is configured by a microcomputer, at least a portion of the storage 110b may be configured as an internal memory of the microcomputer or may be configured as an independent memory.

The input machine 110c is capable of inputting various parameters related to the control of the robot system 100, other types of data, or the like to the arithmetic processor 110a. The input machine 110c is configured by a known input device such as a keyboard, a touch panel, and a button switch group. In the first embodiment, for example, the position information of the patient may be set to be inputtable by the input machine 110c. Further, the position information of the patient may be inputtable by the operating machine 102.

The controller 110 may be configured by a single controller 110 for centralized control, or may be configured by a plurality of controllers 110 that cooperate with each other to perform distributed control. Further, the controller 110 may be configured by a microcomputer, or may be configured by an MPU, a programmable logic controller (PLC), a logic circuit, or the like.

### [Operations, and Actions and Effects of Robot System]

Next, the operation and the action and effect of the robot system 100 according to the first embodiment will be described in detail with reference to FIGS. 1 to 10. The following operations are performed by the arithmetic processor 110a of the controller 110 reading the program stored in the storage 110b.

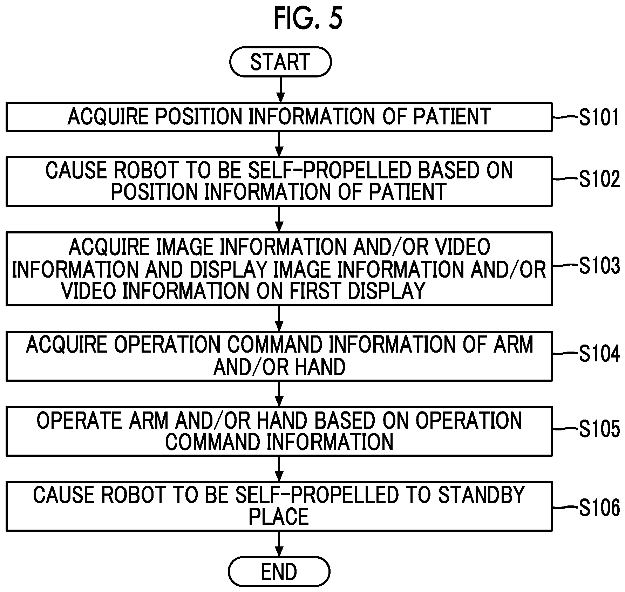

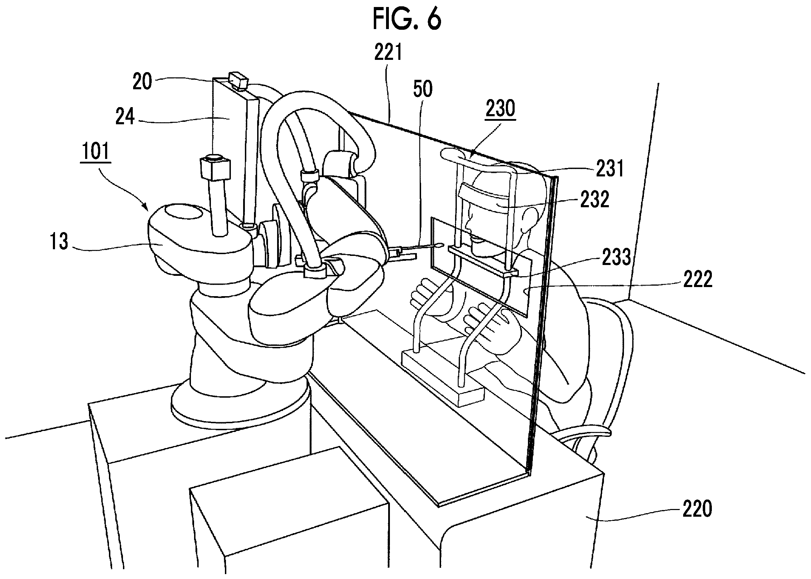

FIG. 5 is a flowchart illustrating an example of the operation of the robot system according to the first embodiment. FIG. 6 is a schematic diagram illustrating an example of the operation of the robot in the robot system according to the first embodiment.

As illustrated in FIG. 5, when the operator operates the input machine 110c (and/or the operating machine 102), the controller 110 acquires the position information of a patient from the input machine 110c (and/or the operating machine 102) (Step S101) .

Then, the controller 110 causes the robot 101 to be self-propelled (automatically moved) from a standby place set in advance to the vicinity of the patient based on the position information of the patient acquired in Step S101 (Step S102) .

Specifically, the controller 110 outputs the position information of the patient acquired in Step S101 to the controller 14. The controller 14 drives the drive motor based on the input position information of the patient and the map information in the hospital stored in the storage, and causes the robot 101 to be self-propelled to the vicinity of the patient.

The standby place may be a place (space) isolated from the first space 201 and the second space 202.

Then, the controller 110 acquires image information and/or video information obtained by picking-up of the first imager 20 and displays the acquired image information and/or video information on the first display 103 (Step S103). The controller 110 may execute the process of Step S103 before the process of Step S101 or Step S102.

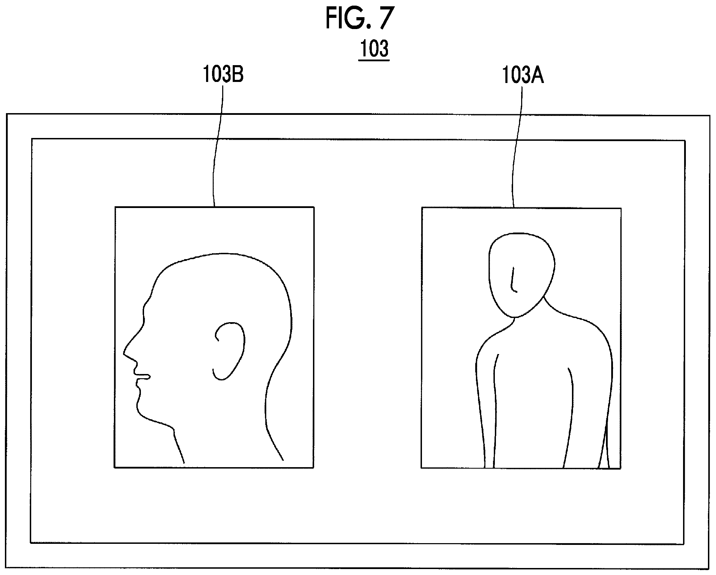

Here, the image information and/or the video information displayed on the first display 103 will be described with reference to FIGS. 7 to 10.

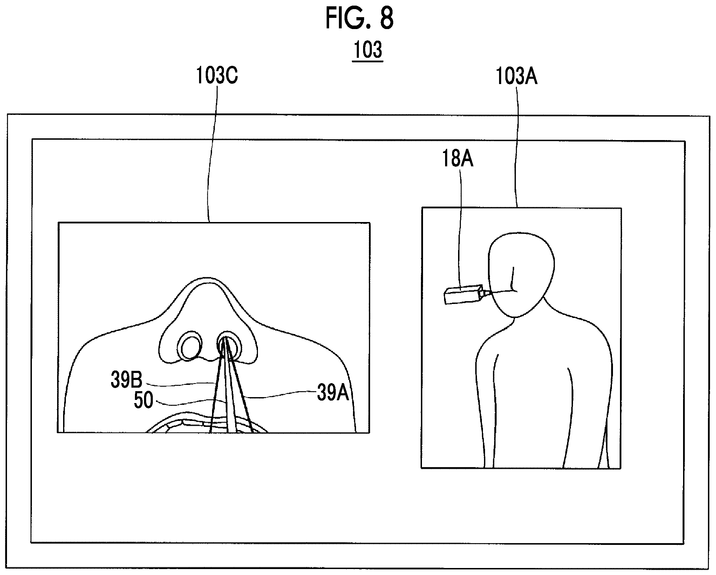

FIG. 7 is a schematic diagram illustrating an example of the image information and/or the video information displayed on the first display 103 illustrated in FIG. 1. FIGS. 8 to 10 are schematic diagrams illustrating other examples of the image information and/or the video information displayed on the first display 103 illustrated in FIG. 1. In FIGS. 8 to 10, illustrations of some portions of the robot 101 and the first hand 18A are omitted.

As illustrated in FIG. 7, video information obtained by picking-up of the first imager 20 (video information obtained by picking up an image of the front of the patient) may be displayed on the first display 103 as first video information 103A. Further, video information obtained by picking-up of an image pickup device (not illustrated) that picks up the profile of the patient may be displayed on the first display 103 as second video information 103B.

Further, as illustrated in FIG. 8, video information obtained by picking-up of the first imager 20 may be displayed on the first display 103 as the first video information 103A. Further, video information obtained by picking-up of the camera 36 provided at the first hand 18A may be displayed on the first display 103 as third video information 103C.

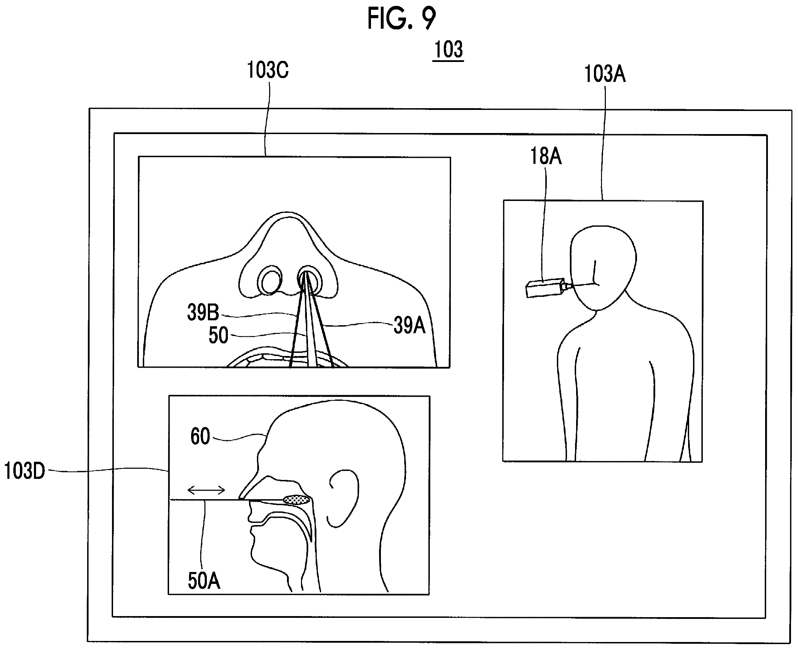

Further, as illustrated in FIG. 9, video information obtained by picking-up of the first imager 20 may be displayed on the first display 103 as the first video information 103A. Further, video information obtained by picking-up of the camera 36 provided at the first hand 18A may be displayed on the first display 103 as third video information 103C.

Further, a virtual model showing the position information of a medical test instrument and/or a medical examination instrument may be displayed on the first display 103 as fourth video information 103D. Specifically, a virtual sterilized cotton swab 50A, which is a virtual model of the sterilized cotton swab 50, and a virtual patient 60, which is a virtual model of the medical target site of the patient, are displayed as the fourth video information 103D.

At this time, the controller 110 may move the virtual sterilized cotton swab 50A in the fourth video information 103D based on the position information of the patient, and position information detected by a rotation sensor that detects a rotation position of each drive motor and/or operation information input to the operating machine 102. This makes it possible for the operator to easily understand the distance between the patient, and the distal end portion of the medical test instrument and/or the medical examination instrument (sterilized cotton swab 50).

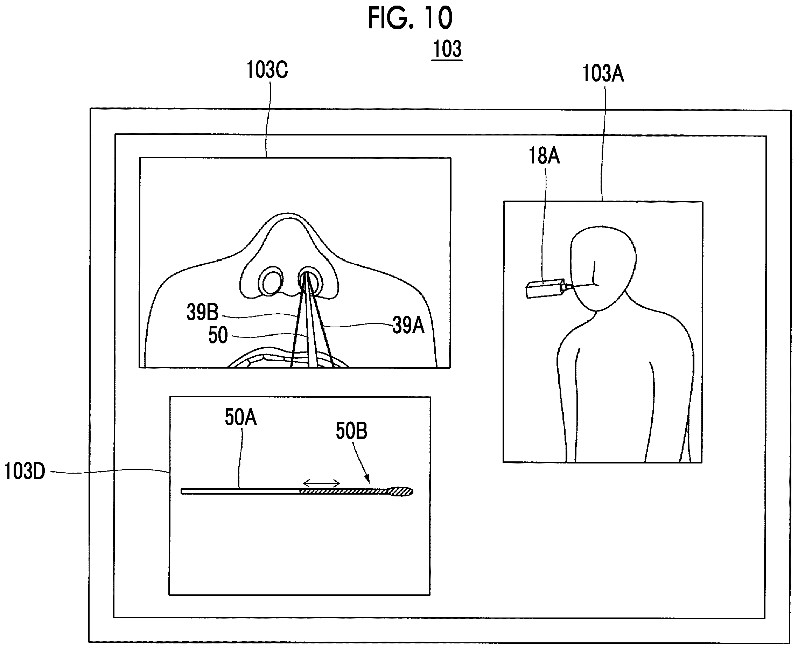

In addition, as illustrated in FIG. 10, video information obtained by picking-up of the first imager 20 may be displayed on the first display 103 as the first video information 103A. Further, video information obtained by picking-up of the camera 36 provided at the first hand 18A may be displayed on the first display 103 as third video information 103C.

Further, a virtual model showing the position information of the medical test instrument and/or the medical examination instrument may be displayed on the first display 103 as the fourth video information 103D. Specifically, as the fourth video information 103D, a virtual sterilized cotton swab 50A, which is a virtual model of the sterilized cotton swab 50, is displayed.

At this time, the controller 110 may display a region of the sterilized cotton swab 50 put into the body of the patient, as a first region 50B in the fourth video information 103D, based on the position information of the patient, and the position information detected by the rotation sensor that detects the rotation position of each drive motor and/or the operation information input to the operating machine 102. The first region 50B may be indicated by hatching, for example, as illustrated in FIG. 10, or may be indicated by a color different from the color of the virtual sterilized cotton swab 50A.

This makes it possible for the operator to easily understand the distance between the patient, and the distal end portion of the medical test instrument and/or the medical examination instrument (sterilized cotton swab 50) .

Then, as illustrated in FIG. 5, the controller 110 acquires the operation command information of the arm 13 and/or the hand 18 from the operating machine 102 (Step S104) . Then, the controller 110 operates the arm 13 and/or the hand 18 based on the operation command information acquired in Step S104 (Step S105) .

As a result, the operator can operate the robot 101 by a remote operation to perform medical practice (for example, examination and/or test) on the patient. For example, the operator may perform work of collecting a specimen for a PCR test from the patient.

Here, the medical practice work by the robot 101 will be described with reference to FIG. 6. In FIG. 6, a form in which the robot 101 includes a second display 24, which will be described later is adopted.

As illustrated in FIG. 6, a shielding plate 221 is disposed between the robot 101 and the patient. The shielding plate 221 may be installed on a base 220 such as a desk. Further, the shielding plate 221 is made of a transparent member such as a glass plate, and an opening 222 is provided in a substantially central portion.

The position and size of the opening 222 are appropriately set in accordance with the type of medical practice. For example, when medical practice for internal medicine, otolaryngology, or the like is performed, the disposition position and size of the opening 222 are appropriately set so that the mouth and the nose (medical practice target portion) of the patient are located at the opening 222. Further, when medical practice related to ophthalmology is performed, the disposition position and size of the opening 222 are appropriately set so that the eye (medical practice target portion) of the patient is located at the opening 222.

As a result, it is possible to suppress an occurrence of a situation in which, when the patient coughs or sneezes, the droplets adhere to the robot 101.

Further, a positioning device 230 is installed between the shielding plate 221 and the patient. The positioning device 230 includes a main body 231, an abutting target portion 232, and a chin rest 233. The main body 231 may be configured such that the patient can grip the main body 231. Further, the chin rest 233 may be configured to move up and down.

In the positioning device 230, the patient abuts the forehead on the abutting target portion 232 and puts the chin on the chin rest 233, thereby the medical practice target portion of the patient is positioned within a range (opening 222) set in advance. This facilitates the positioning of the medical practice target portion of the patient, and thus makes it possible to reduce the burden on the operation of the operator.

When executing the process of Step S105, the controller 110 may automatically operate the arm 13 and/or the hand 18 so that, for example, the distal end portion of the medical test instrument or medical examination instrument held by the hand 18 approaches the patient.

Further, the controller 110 may store the operation command information of the arm 13 and/or the hand 18 input from the operating machine 102, in the storage 110b. Further, the controller 110 may operate the arm 13 and/or the hand 18 based on the operation command information stored in the storage 110b, to perform medical practice (for example, examination work and/or test work) on the patient.

Further, the controller 110 may be configured to learn the examination work and the like. Specifically, for example, when the controller 110 causes the robot 101 to perform examination work or the like, in a case where the operator operates the operating machine 102 to correct the operation of the arm 13 and/or the hand 18, the controller 110 stores the corrected operation command information of the arm 13 and/or the hand 18 in the storage 110b.

Then, the controller 110 operates the arm 13 and/or the hand 18 based on the corrected operation command information to perform medical practice (for example, examination work and/or test work) on the patient. Then, when the operation of the arm 13 and/or the hand 18 is corrected again by the operator, the controller 110 stores the corrected operation command information of the arm 13 and/or the hand 18 in the storage 110b, and learns the examination work and the like.

Then, when the operator operates the operating machine 102 (and/or the input machine 110c), and thus a medical practice termination command is input from the operating machine 102 (and/or the input machine 110c), the controller 110 causes the robot 101 to be self-propelled to the standby place (Step S106), and then terminates this program.

The controller 110 may control the robot 101 to be in a standby state after the robot 101 is self-propelled to the standby place and then disinfected by appropriate means. Further, the robot 101 may be disinfected by a worker wearing a protective mask and protective clothing.

In the robot system 100 according to the first embodiment, which is configured in this manner, the robot 101 is self-propelled to the vicinity of the patient only by the operator (medical staff) inputting the position information of the patient. As a result, the operator can concentrate on the medical practice, and thus it is possible to reduce the burden of the operation of the operator.

Further, in the robot system 100 according to the first embodiment, the operator operates the robot 101 in the second space 202 isolated from the patient.

This makes it possible to suppress a contact between the operator and a patient suspected of being infected with a virus or the like. Therefore, it is possible to sufficiently suppress the infection to the operator with a virus or the like.

Further, since the robot 101 moves to the vicinity of the patient in each hospitalization room or the like, the operator does not need to move in the hospital. Therefore, it is possible to sufficiently suppress the infection to the operator.

Further, since the robot 101 moves, it is possible to reduce the number of movements and/or the movement distance of the patient infected with a virus or the like in the hospital. This makes it possible to reduce the spread of viruses and the like.

Further, in the robot system 100 according to the first embodiment, the pair of laser pointers 38A and 38B are disposed at the first hand 18A (hand 18) so that the rays of laser light 39A and 39B with which irradiation is respectively performed by the pair of laser pointers 38A and 38B intersect with each other.

As a result, when the first hand 18A approaches the patient, a distance between the laser light 39A and the laser light 39B that hit the patient becomes smaller. Further, when the first hand 18A approaches the patient, the laser light that hits the patient becomes one point. Further, when the first hand 18A approaches the patient, the distance between the laser light 39A and the laser light 39B that hit the patient increases.

Therefore, the operator (medical staff) can easily understand a distance between the patient, and the distal end portion of the medical test instrument and/or the medical examination instrument (sterilized cotton swab 50) by the rays of laser light 39A and 39B with which irradiation is performed from the pair of laser pointers 38A and 38B.

Further, in the robot system 100 according to the first embodiment, the virtual model showing the position information of the medical test instrument and/or the medical examination instrument is displayed on the first display 103 as the fourth video information 103D. This makes it possible for the operator to easily understand the distance between the patient, and the distal end portion of the medical test instrument and/or the medical examination instrument (sterilized cotton swab 50).

At this time, the controller 110 displays the virtual patient 60, which is a virtual model of the medical target site of the patient, on the first display 103, thereby it is possible to more easily understand the distance between the patient, and the distal end portion of the medical test instrument and/or the medical examination instrument (sterilized cotton swab 50).

### [First Modification Example]

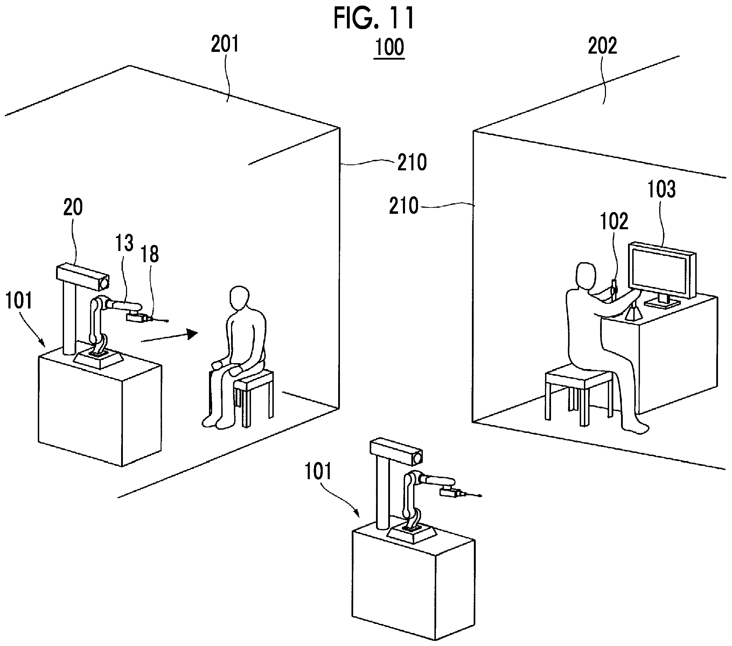

Next, a first modification example of the robot system 100 according to the first embodiment will be described below with reference to FIG. 11.

FIG. 11 is a schematic diagram illustrating a schematic configuration of a robot system according to the first modification example in the first embodiment.

As illustrated in FIG. 11, the robot system 100 in the first modification example has the same basic configuration as the robot system 100 according to the first embodiment, but the first modification example is different from the first embodiment in that a robot 101 is configured by a vertical articulated robot.

The robot system 100 in the first modification example, which is configured as described above, also has the similar action and effect to those of the robot system 100 according to the first embodiment.

### (Second Embodiment)

In a robot system according to a second embodiment, a robot further includes a first audio input and a first audio output in the robot system according to the first embodiment (including the first modification example). In addition, a second audio input and a second audio output are further disposed in a second space. A controller outputs audio information input to the first audio input, to the second audio output, and outputs audio information input to the second audio input, to the first audio output.

In a control method for a robot system according to the second embodiment, the robot further includes the first audio input and the first audio output in the control method for the robot system according to the first embodiment (including the first modification example). In addition, a second audio input and a second audio output are further disposed in a second space. A controller outputs audio information input to the first audio input, to the second audio output, and outputs audio information input to the second audio input, to the first audio output.

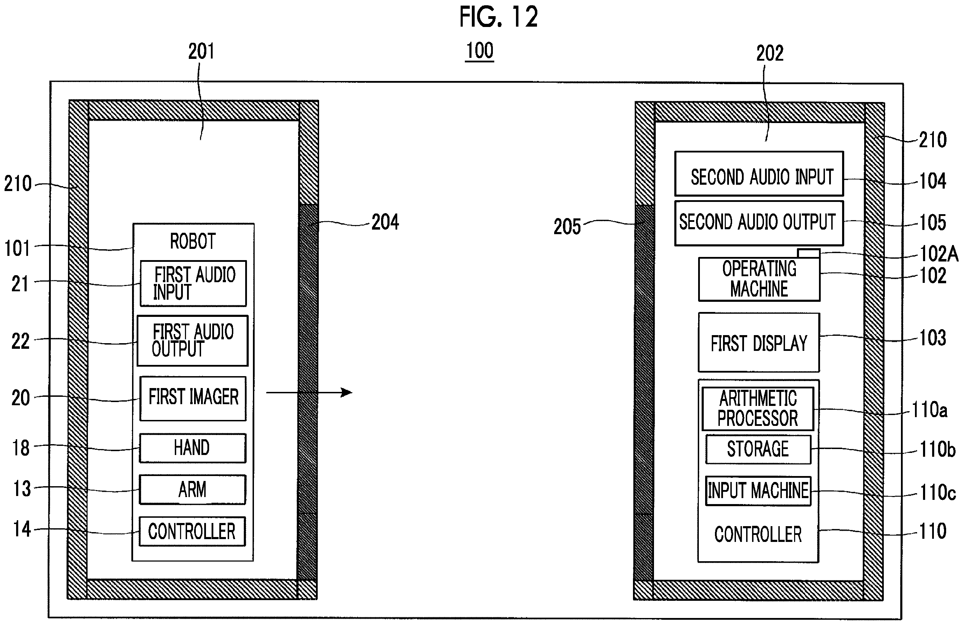

An example of the robot system according to the second embodiment will be described below with reference to FIG. 12.

### [Configuration of Robot System]

FIG. 12 is a schematic diagram illustrating a schematic configuration of the robot system according to the second embodiment.

As illustrated in FIG. 12, the robot system 100 according to the second embodiment has the same basic configuration as the robot system 100 according to the first embodiment. The second embodiment is different from the first embodiment in that the robot 101 includes a first audio input 21 and a first audio output 22, and that a second audio input 104 and a second audio output 105 are disposed in the second space 202.

The first audio input 21 and the second audio input 104 may be configured by, for example, a microphone. Further, the first audio output 22 and the second audio output 105 may be configured by a speaker.

The second audio input 104 and the second audio output 105 may be configured by headphones (headsets) with a microphone. When the first display 103 is configured by a head-mounted display, the second audio input 104 and the second audio output 105 may be configured by a microphone and headphones attached to the head-mounted display.

The robot system 100 in the second embodiment, which is configured as described above, also has the similar action and effect to those of the robot system 100 according to the first embodiment.

Further, in the robot system 100 according to the second embodiment, the robot 101 is provided with the first audio input 21 and the first audio output 22, and the second audio input 104 and the second audio output 105 are disposed in the second space 202. Thus, it is possible to perform communication between the patient and the operator.

As a result, for example, when the operator is a medical staff, or when there is a medical staff beside the operator, it is possible to perform, on the patient, medical practice such as inquiries, auscultation, transmission of test results, communication of treatment policies, and the like.

### (Third Embodiment)

In a robot system according to a third embodiment, a robot further includes a container that contains at least one transfer item among a drug, a meal, a test reagent (reagent), a specimen, a medical test instrument, and a medical examination instrument in the robot system according to the first embodiment (including the first modification example) or the second embodiment.

In a control method for a robot system according to the third embodiment, the robot further includes the container that contains at least one transfer item among a drug, a meal, a test reagent (reagent), a specimen, a medical test instrument, and a medical examination instrument in the control method for the robot system according to the first embodiment (including the first modification example) or the second embodiment.

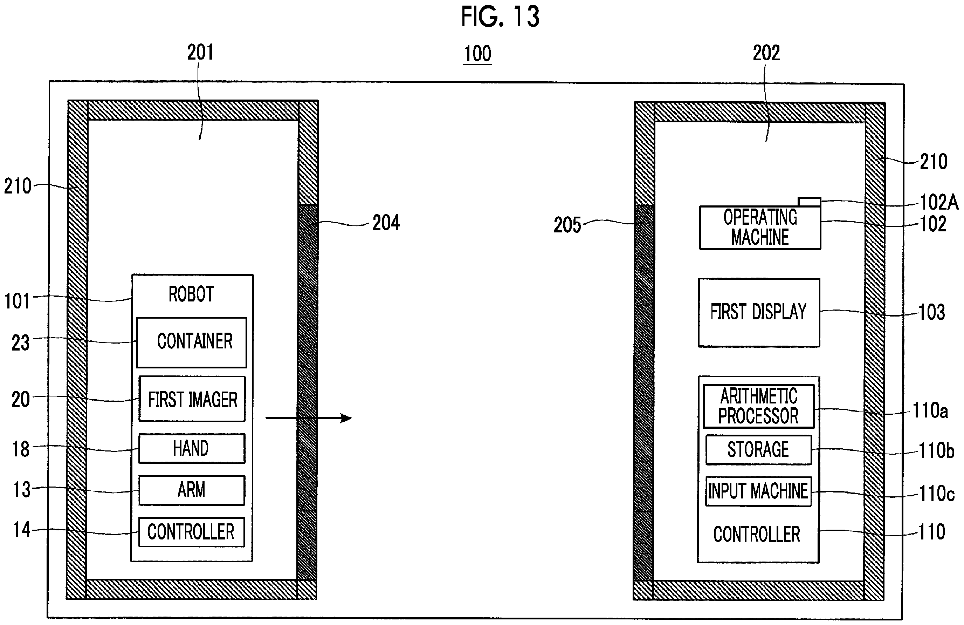

An example of the robot system according to the third embodiment will be described below with reference to FIG. 13.

### [Configuration of Robot System]

FIG. 13 is a schematic diagram illustrating a schematic configuration of the robot system according to the third embodiment.

As illustrated in FIG. 13, a robot system 100 according to the third embodiment has the same basic configuration as the robot system 100 according to the second embodiment. The third embodiment is different from the second embodiment in that a robot 101 further includes a container 23 that contains at least one transfer item among a drug, a meal, a test reagent, a specimen, a medical test instrument, and a medical examination instrument.

As the container 23, various containment items such as a box with a lid and a tray can be used. Further, the container 23 may be made of metal (for example, stainless steel) so as to be capable of supporting a sterilization process such as autoclave sterilization and dry heat sterilization. Further, the container 23 may be configured so that the internal space can be maintained at a predetermined temperature (for example, 0°C, -20°C, or - 80°C) in order to be able to transfer a specimen.

Further, various instruments and/or experimental devices such as an autopipette, a tip used for the autopipette, a microtube, a centrifuge settling tube, a centrifuge, and a PCR device may be contained in the container 23.

The robot system 100 in the third embodiment, which is configured as described above, also has the similar action and effect to those of the robot system 100 according to the first embodiment.

Further, in the robot system 100 according to the third embodiment, the robot 101 further includes the container 23 that contains at least one transfer item among a drug, a meal, a test reagent, a specimen, a medical test instrument, and a medical examination instrument. This makes it possible to reduce the work of medical practice assistants such as nurses. In addition, since it is possible to reduce the chance of the medical practice assistant coming into contact with a patient with an infectious disease such as a virus, it is possible to sufficiently suppress the infection of the medical practice assistant with a virus or the like.

### (Fourth Embodiment)

In a robot system according to a fourth embodiment, a third space isolated from the first space and the second space is further provided in the robot system according to any of the first embodiment (including the first modification example) to the third embodiment. In addition, a robot is disinfected in the third space.

Further, in the robot system according to the fourth embodiment, the robot may be configured to disinfect the robot itself.

Further, in the robot system according to the fourth embodiment, a controller may be configured to further perform (C) self-propelling of the robot to the third space and disinfection of the robot, after (B).

In a control method for a robot system according to a fourth embodiment, a third space isolated from the first space and the second space is further provided in the control method for the robot system according to any of the first embodiment (including the first modification example) to the third embodiment. In addition, a robot is disinfected in the third space.

Further, in the control method for the robot system according to the fourth embodiment, the robot may be configured to disinfect the robot itself.

Further, the control method for the robot system according to the fourth embodiment may further include (C) in which the robot is self-propelled to the third space and disinfects the robot after (B).

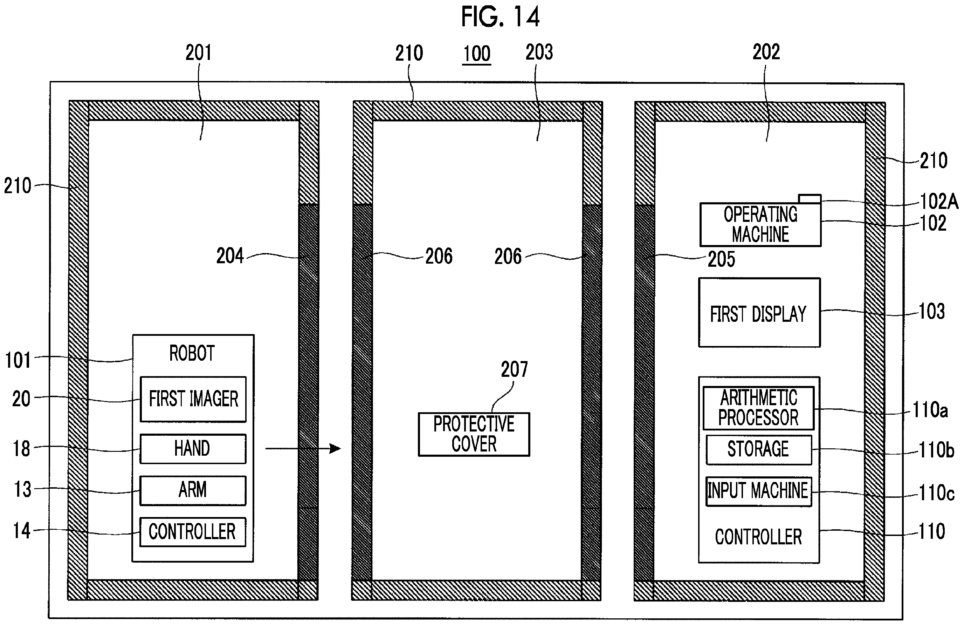

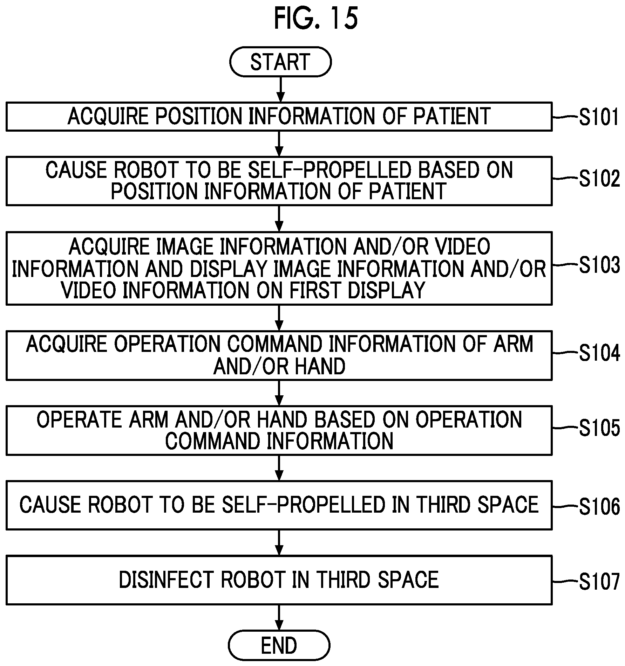

An example of the robot system according to the fourth embodiment will be described below with reference to FIGS. 14 and 15.

### [Configuration of Robot System]

FIG. 14 is a schematic diagram illustrating a schematic configuration of the robot system according to the fourth embodiment.

As illustrated in FIG. 14, a robot system 100 according to the fourth embodiment has the same basic configuration as the robot system 100 according to the first embodiment. The fourth embodiment is different from the first embodiment in that a third space 203 isolated from the first space 201 and the second space 202 are further provided.

The first space 201, the second space 202, and the third space 203 are spaces separated from each other. The first space 201, the second space 202, and the third space 203 are separated by a partition wall member 210, respectively.

An anterior chamber may be provided in a room (sterility chamber) constituting the third space 203. Further, a fan filter unit that makes the anterior chamber have negative pressure and makes the second space 202 (internal space of the sterility chamber) have positive pressure may be installed in the anterior chamber. A known fan filter unit can be used as the fan filter unit.

The partition wall member 210 may be provided with a shutter (door) 206 that permits/prohibits movement into the third space 203.

The robot 101 may be configured to disinfect the robot 101 itself. Specifically, the robot 101 may be disinfected by itself, for example, in a manner that a sprayer for spraying a solution such as an ethanol solution having sterilizing and antiviral effects is held with the hand 18 and the solution is sprayed toward the robot 101.

Further, the robot 101 may be disinfected by itself in a manner that an irradiator for irradiation with ultraviolet rays is held with the hand 18, and the robot 101 is irradiated with the ultraviolet rays.

Further, a protective cover 207 (surgical drape) may be disposed in the third space 203. The robot 101 may be configured to maintain a sterilized/antiviral state by detachably attaching the protective cover 207 (by the robot 101 being covered with the protective cover 207).

Specifically, the robot 101 wears the protective cover 207 in the third space 203, and then moves into the first space 201 to perform medical practice. After the medical practice is terminated, the robot 101 moves into another third space in which the protective cover 207 is not disposed, and then removes the protective cover 207. Then, the robot 101 moves into the third space 203 in which the protective cover 207 is disposed, and wears the protective cover 207.

### [Operations, and Actions and Effects of Robot System]

Next, the operation and the action and effect of the robot system 100 according to the fourth embodiment will be described in detail with reference to FIGS. 14 and 15. The following operations are performed by the arithmetic processor 110a of the controller 110 reading the program stored in the storage 110b.

FIG. 15 is a flowchart illustrating an example of the operation of the robot system according to the fourth embodiment.

As illustrated in FIG. 15, the operation of the robot system 100 according to the fourth embodiment is basically the same as that of the robot system 100 according to the first embodiment. The fourth embodiment is different from the first embodiment in that the controller 110 executes a process of Step S106A instead of the process of Step S106, and executes a process of Step S107 after the process of Step S106A.

Specifically, when medical practice termination command information is input from the operating machine 102 (and/or the input machine 110c), the controller 110 causes the robot 101 to be self-propelled to the third space 203 (Step S106A).

Then, the controller 110 disinfects the robot 101 in the third space 203 (Step S107), and terminates this program.

The robot system 100 in the fourth embodiment, which is configured as described above, also has the similar action and effect to those of the robot system 100 according to the first embodiment.

Further, in the robot system 100 according to the fourth embodiment, the robot 101 disinfects the robot 101 itself. This eliminates the need for the worker wearing the protective mask and the protective clothing to disinfect the robot 101. Therefore, it is possible to provide an easy-to-use robot system 100.

### [First Modification Example]

Next, a first modification example of the robot system 100 according to the fourth embodiment will be described.

In a robot system according to the first modification example in the fourth embodiment, a disinfector that disinfects the robot is disposed in the third space.

In a control method for a robot system according to the first modification example in the fourth embodiment, a disinfector that disinfects the robot is disposed in the third space.

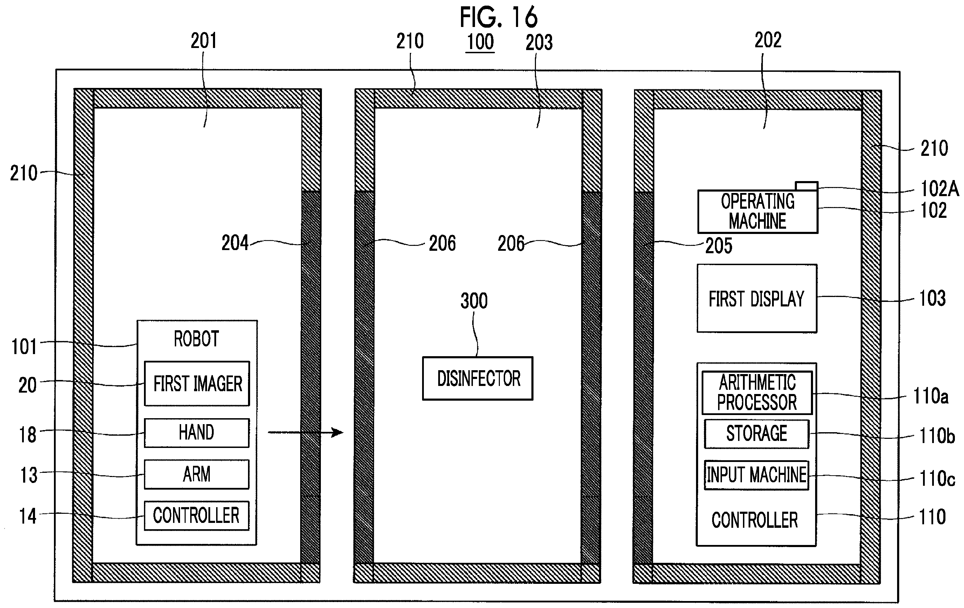

An example of the robot system according to the first modification example in the fourth embodiment will be described below with reference to FIG. 16.

### [Configuration of Robot System]

FIG. 16 is a schematic diagram illustrating a schematic configuration of a robot system according to the first modification example in the fourth embodiment.

As illustrated in FIG. 16, the robot system 100 in the first modification example has the same basic configuration as that of the robot system 100 according to the fourth embodiment. The first modification example is different from the fourth embodiment in that a disinfector 300 is disposed in a sterility chamber constituting the third space 203.

The disinfector 300 may be a sprayer that sprays a solution such as an ethanol solution having sterilizing and antiviral effects. Further, the disinfector 300 may be an irradiator that performs irradiation with ultraviolet rays. Further, a robot different from the robot 101 may be disposed in the sterility chamber, and the robot may hold the sprayer or the irradiator to perform the disinfection work on the robot 101.

The robot system 100 in the first modification example, which is configured as described above, also has the similar action and effect to those of the robot system 100 according to the fourth embodiment.

### (Fifth Embodiment)

In a robot system according to a fifth embodiment, a robot further includes a second display in the robot system according to any of the first embodiment to the fourth embodiment (including the modification examples). In addition, a second imager is further disposed in the second space, the controller displays image information and/or video information obtained by picking-up of the second imager, on the second display in (B).

In a control method for a robot system according to the fifth embodiment, a robot further includes a second display in the control method for the robot system according to any of the first embodiment to the fourth embodiment (including the first modification examples). In addition, a second imager is further disposed in the second space, and, in (B), the second display displays image information and/or video information obtained by picking-up of the second imager.

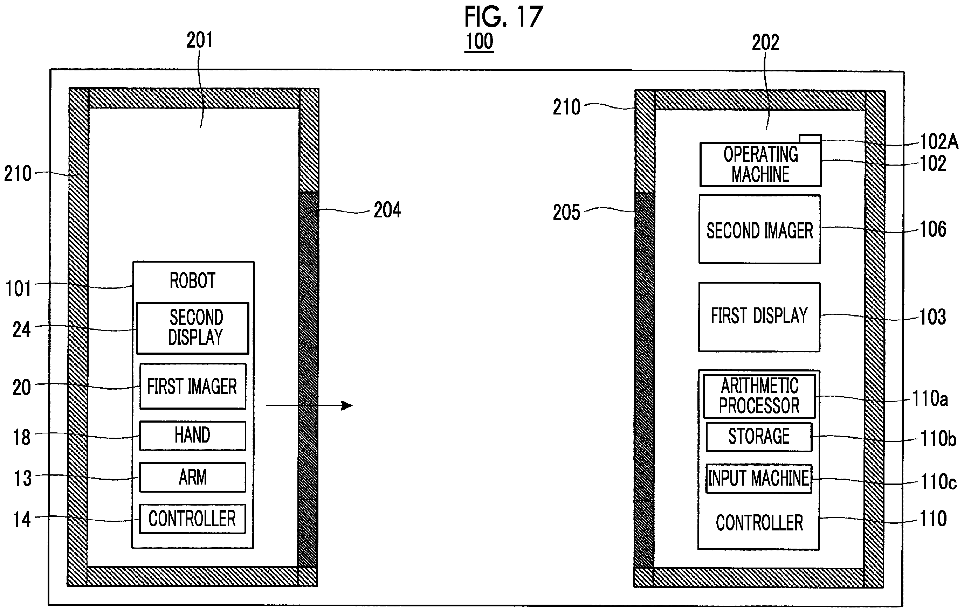

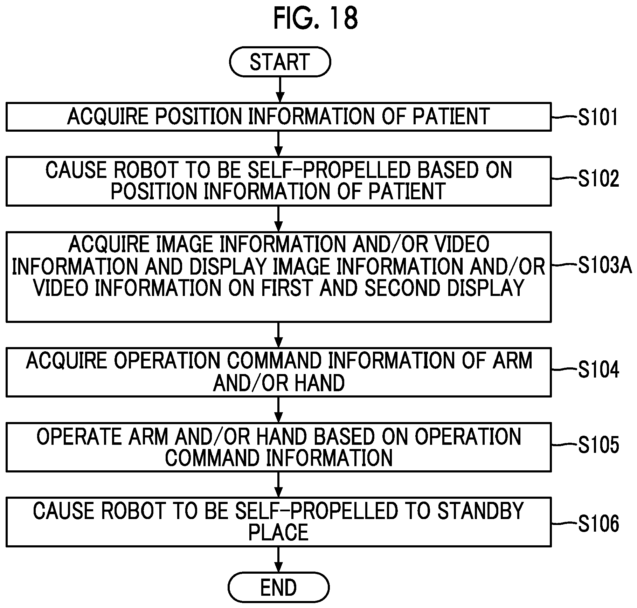

An example of the robot system according to the fifth embodiment will be described below with reference to FIGS. 17 and 18.

### [Configuration of Robot System]

FIG. 17 is a schematic diagram illustrating a schematic configuration of the robot system according to the fifth embodiment.

As illustrated in FIG. 17, a robot system 100 according to the fifth embodiment has the same basic configuration as that of the robot system 100 according to the first embodiment. The fifth embodiment is different from the first embodiment in that a robot 101 further includes a second display 24, and that a second imager 106 is further disposed in the second space 202.

The second display 24 displays image information and/or video information obtained by picking-up of the second imager 106. The second display 24 may be configured by, for example, a stationary type display.

The second imager 106 picks up an image and/or a video, and outputs the image information and/or video information obtained by pickup to the second display 24 via the controller 110 and the controller 14. The second imager 106 may be, for example, a video camera.

### [Operations, and Actions and Effects of Robot System]

Next, the operation and the action and effect of the robot system 100 according to the fifth embodiment will be described in detail with reference to FIGS. 17 and 18. The following operations are performed by the arithmetic processor 110a of the controller 110 reading the program stored in the storage 110b.

FIG. 18 is a flowchart illustrating an example of the operation of the robot system according to the fifth embodiment.

As illustrated in FIG. 18, the operation of the robot system 100 according to the fifth embodiment is basically the same as that of the robot system 100 according to the first embodiment. The fifth embodiment is different from the first embodiment in that the controller 110 executes a process of Step S103A instead of the process of Step S103.

Specifically, the controller 110 is self-propelled from the standby place to the vicinity of the patient (Step S102), and then executes processes as follows.

The controller 110 acquires the image information and/or the video information obtained by picking-up of the first imager 20, and displays the image information and/or the video information on the first display 103. In addition, the controller 110 acquires the image information and/or the video information obtained by picking-up of the second imager 106, and displays the image information and/or the video information on the second display 24 (Step S103A). The controller 110 may execute the process of Step S103A before the process of Step S101 or Step S102.

The robot system 100 in the fifth embodiment, which is configured as described above, also has the similar action and effect to those of the robot system 100 according to the first embodiment.

Further, in the robot system 100 according to the fifth embodiment, the robot 101 further includes the second display 24, and the second imager 106 is further disposed in the second space 202.

This makes it possible to communicate between the patient and the operator (medical staff).

### (Sixth Embodiment)

A robot system according to a sixth embodiment includes a robot including an arm with a hand that holds a medical test instrument and/or a medical examination instrument, an operating machine that operates the robot, and a controller. A first space in which the robot is disposed is isolated from a second space in which the operating machine is disposed. A first imager is disposed at the hand. The controller performs (α) to operate the arm and/or the hand based on operation command information of the arm and/or the hand, which is input from the operating machine.

Further, the robot system according to the sixth embodiment may further include a first imager and a first display that displays image information and/or video information obtained by picking-up of the first imager. The controller may be configured to perform (α) in a state where the first display displays the image information and/or the video information obtained by picking-up of the first imager.

Further, in the robot system according to the sixth embodiment, the robot and the operating machine may be configured by a master-slave method.

Further, in the robot system according to the sixth embodiment, the first imager may be disposed at the robot (a portion other than the hand), or may be disposed at the hand of the robot.

Further, in the robot system according to the sixth embodiment, a pair of laser light indicators may be disposed at the hand so that the rays of light with which irradiation is performed from the laser light indicators intersect with each other.

Further, in the robot system according to the sixth embodiment, the first display may be configured to display a virtual model showing position information of the medical test instrument and/or the medical examination instrument.

Further, in the robot system according to the sixth embodiment, the first display may be configured to display a virtual model of a medical practice target site of a patient.

Further, in the robot system according to the sixth embodiment, an operation switch for an instruction to release holding the medical test instrument and/or the medical examination instrument may be disposed in the operating machine.

Further, in a control method for a robot system according to a sixth embodiment, a robot system includes a robot including an arm with a hand that holds a medical test instrument and/or a medical examination instrument, and an operating machine that operates the robot. A first space in which the robot is disposed is isolated from a second space in which the operating machine is disposed. A first imager is disposed at the hand. The control method includes (α) in which the arm and/or the hand operates based on operation command information of the arm and/or the hand, which is input from the operating machine.

Further, in the control method for the robot system according to the sixth embodiment, the robot system may further include a first imager and a first display that displays image information and/or video information obtained by picking-up of the first imager. (α) may be performed in a state where the first display displays the image information and/or the video information obtained by picking-up of the first imager.

Further, in the control method for the robot system according to the sixth embodiment, the robot and the operating machine may be configured by a master-slave method.

Further, in the control method for the robot system according to the sixth embodiment, the first imager may be disposed at the robot or at the hand.

Further, in the control method for the robot system according to the sixth embodiment, a pair of laser light indicators may be disposed at the hand so that the rays of light with which irradiation is performed from the laser light indicators intersect with each other.

Further, in the control method for the robot system according to the sixth embodiment, the first display may be configured to display a virtual model showing position information of the medical test instrument and/or the medical examination instrument.

Further, in the control method for the robot system according to the sixth embodiment, the first display may be configured to display a virtual model of a medical practice target site of a patient.

Further, in the control method for the robot system according to the sixth embodiment, an operation switch for an instruction to release holding the medical test instrument and/or the medical examination instrument may be disposed at the operating machine.

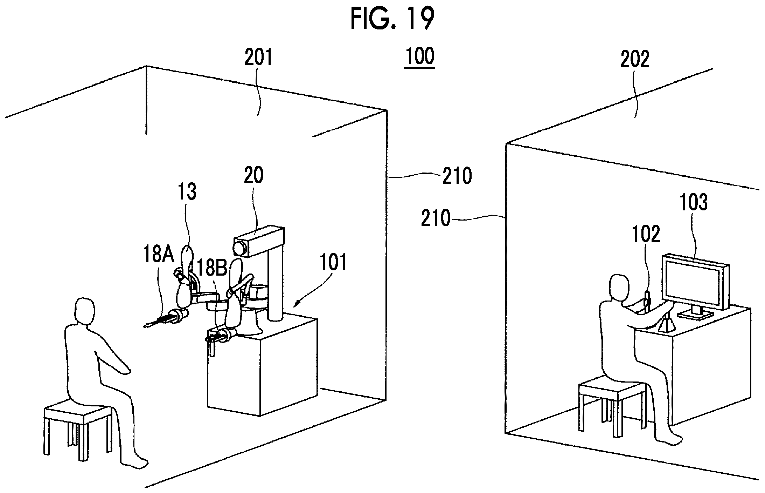

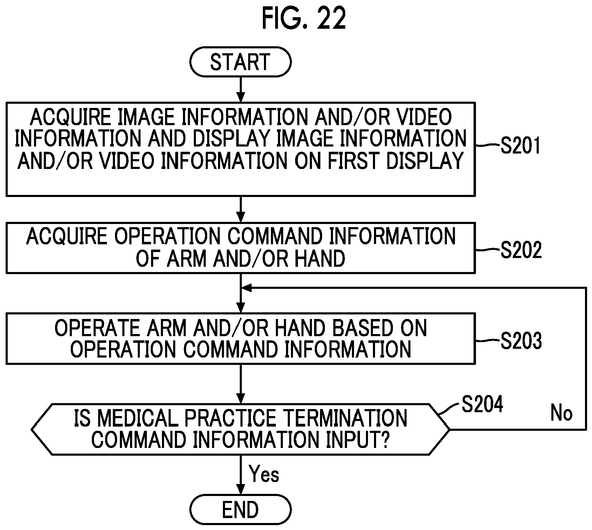

An example of the robot system according to the sixth embodiment will be described below with reference to FIGS. 19 and 22.

### [Configuration of Robot System]

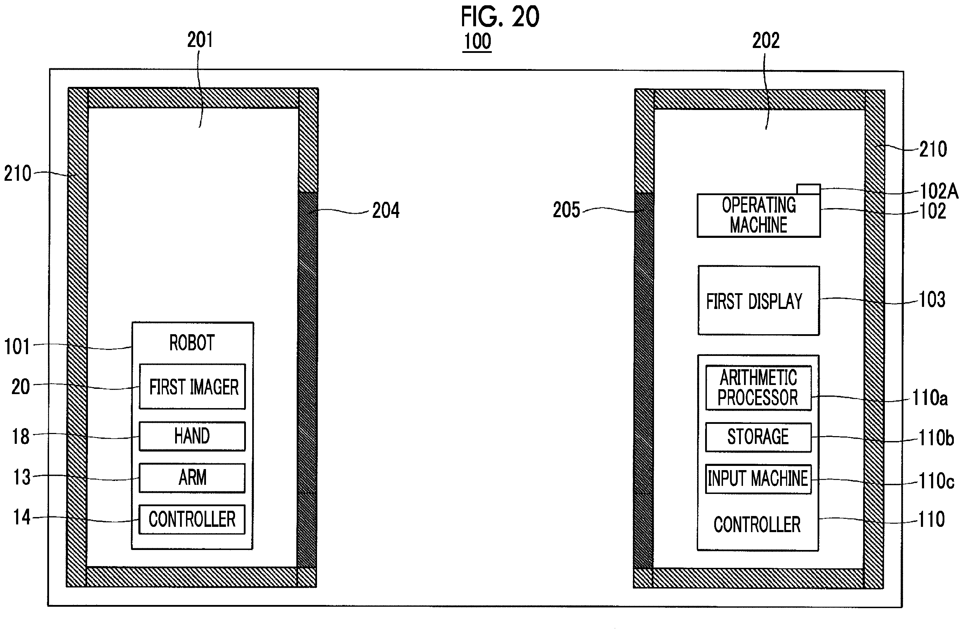

FIGS. 19 and 20 are schematic diagrams illustrating a schematic configuration of the robot system according to the sixth embodiment.

As illustrated in FIGS. 19 and 20, a robot system 100 according to the sixth embodiment has the same basic configuration as that of the robot system 100 according to the first embodiment. The sixth embodiment is different from the first embodiment in that a robot 101 is installed in a first space 201 (the robot 101 is a stationary type). Further, the configuration of a first hand 18A in the robot 101 is different.

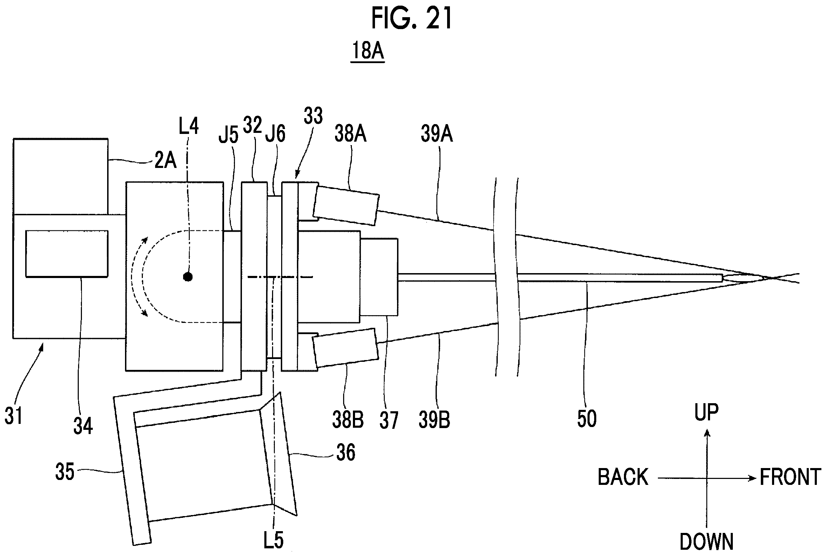

Here, the configuration of the first hand 18A of the robot 101 will be described with reference to FIG. 21.

FIG. 21 is a schematic diagram illustrating a schematic configuration of the hand of the robot illustrated in FIG. 19. In FIG. 21, an up-down direction and a front-back direction in the robot are represented as an up-down direction and a front-back direction in FIG. 21.

As illustrated in FIG. 21, the first hand 18A includes a main body 31, an intermediate member 32, and a holding member 33. The main body 31 and the intermediate member 32 are joined to each other via a rotating joint J5. Further, the intermediate member 32 and the holding member 33 are joined to each other via a rotating joint J6. As a result, the holding member 33 can rotate around a rotation axis L4 and/or a rotation axis L5 with respect to the main body 31.

The main body 31 is provided with an actuator 34 for rotating the holding member 33. The actuator 34 may be, for example, a servomotor servo-controlled by the controller 14. Further, the main body 31 is provided with a rotation sensor (not illustrated) that detects the rotation position of the servomotor and a current sensor (not illustrated) that detects a current for controlling the rotation of the servomotor. The rotation sensor may be, for example, an encoder. Position information detected by the rotation sensor and current information detected by the current sensor may be output to the controller 110 via the controller 14.

A support member 35 is provided at the lower end portion of the intermediate member 32. A camera (first imager) 36 is attached to the support member 35. The camera 36 picks up an image and/or a video, and outputs the image information and/or video information obtained by pickup to the controller 110. The camera 36 may be, for example, a video camera or an X-ray image pickup device.

Further, a target picked up by the camera 36 may be the nostril of the patient. For example, when the nasopharyngeal swab is collected with a sterilized cotton swab, the target may be the nostril of the patient. Further, the target picked up by the camera 36 may be, for example, the oral cavity of the patient when saliva or a specimen derived from the lower respiratory tract (sputum or the like) is collected by a suction catheter or the like.

In the sixth embodiment, a form in which the support member 35 and the camera 36 are disposed at the lower end portion of the intermediate member 32 has been adopted, but the present disclosure is not limited to this. The support member 35 and the camera 36 may be disposed at the upper end portion of the intermediate member 32 or the like. Further, the support member 35 and the camera 36 may be disposed at the holding member 33.

A chuck mechanism 37 for holding/releasing (releasing the holding) a medical test instrument or a medical examination instrument is attached to the holding member 33. The chuck mechanism 37 may be configured by, for example, an air chuck. Here, the chuck mechanism 37 holds a sterilized cotton swab 50 for collecting a specimen for a PCR test.

Further, a pair of laser pointers (laser light indicators) 38A and 38B are arranged at the holding member 33. The laser pointers 38A and 38B are disposed so that rays of laser light 39A and 39B checked from the respective laser pointers 38A and 38B intersect with each other in front of the first hand 18A. In addition, three or more laser light indicators may be disposed at the first hand 18A.

As a result, when the first hand 18A approaches the patient, a distance between the laser light 39A and the laser light 39B that hit the patient becomes smaller. Further, when the first hand 18A approaches the patient, the laser light that hits the patient becomes one point. Further, when the first hand 18A approaches the patient, the distance between the laser light 39A and the laser light 39B that hit the patient increases.

Therefore, the operator (medical staff) can easily understand a distance between the patient, and the distal end portion of the medical test instrument and/or the medical examination instrument (sterilized cotton swab 50) by the rays of laser light 39A and 39B with which irradiation is performed from the pair of laser pointers 38A and 38B.

### [Operations, and Actions and Effects of Robot System]

Next, the operation and the action and effect of the robot system 100 according to the sixth embodiment will be described in detail with reference to FIGS. 19 to 22. The following operations are performed by the arithmetic processor 110a of the controller 110 reading the program stored in the storage 110b.

FIG. 22 is a flowchart illustrating an example of the operation of the robot system according to the sixth embodiment.

As illustrated in FIG. 22, the controller 110 acquires image information and/or video information obtained by picking-up of the first imager 20 and displays the acquired image information and/or video information on the first display 103 (Step S201). The image information and/or video information displayed on the first display 103 may be the same as that in the examples illustrated in FIGS. 6 to 10.

Then, the controller 110 acquires operation command information of the arm 13 and/or the hand 18 from the operating machine 102 (Step S202). Then, the controller 110 operates the arm 13 and/or the hand 18 based on the operation command information acquired in Step S202 (Step S203).

As a result, the operator can operate the robot 101 by a remote operation to perform medical practice (for example, examination and/or test) on the patient (see FIG. 6). For example, the operator may perform work of collecting a specimen for a PCR test.

The controller 110 may store the operation command information of the arm 13 and/or the hand 18 input from the operating machine 102, in the storage 110b. Further, the controller 110 may operate the arm 13 and/or the hand 18 based on the operation command information stored in the storage 110b to perform medical practice (for example, examination work and/or test work) on the patient.

Further, the controller 110 may be configured to learn the examination work and the like. Specifically, for example, when the controller 110 causes the robot 101 to perform examination work or the like, in a case where the operator operates the operating machine 102 to correct the operation of the arm 13 and/or the hand 18, the controller 110 stores the corrected operation command information of the arm 13 and/or the hand 18 in the storage 110b.

Then, the controller 110 operates the arm 13 and/or the hand 18 based on the corrected operation command information to perform medical practice (for example, examination work and/or test work) on the patient. Then, when the operation of the arm 13 and/or the hand 18 is corrected again by the operator, the corrected operation command information of the arm 13 and/or the hand 18 is stored in the storage 110b, and the examination work and the like is learned.

Then, when the operator operates the operating machine 102 (and/or the input machine 110c), and thus medical practice termination command information is input from the operating machine 102 (and/or the input machine 110c) (Yes in Step S204), the controller 110 terminates this program.

After terminating this program, the controller 110 may control the robot 101 to be in a standby state after disinfecting the robot 101 by appropriate means. Further, the robot 101 may be disinfected by a worker wearing a protective mask and protective clothing.

In the robot system 100 according to the sixth embodiment, which is configured in this manner, the operator operates the robot 101 in the second space 202 isolated from the patient.

This makes it possible to suppress a contact between the operator (medical staff and the like) and a patient suspected of being infected with a virus or the like. Therefore, it is possible to sufficiently suppress the infection to the operator with a virus or the like.

Further, in the robot system 100 according to the sixth embodiment, the pair of laser pointers 38A and 38B are disposed at the first hand 18A (hand 18) so that the rays of laser light 39A and 39B with which irradiation is respectively performed by the pair of laser pointers 38A and 38B intersect with each other.

Thus, when the first hand 18A approaches the patient, a distance between the laser light 39A and the laser light 39B that hit the patient becomes smaller. Further, when the first hand 18A approaches the patient, the laser light that hits the patient becomes one point. Further, when the first hand 18A approaches the patient, the distance between the laser light 39A and the laser light 39B that hit the patient increases.

Therefore, the operator (medical staff) can easily understand a distance between the patient, and the distal end portion of the medical test instrument and/or the medical examination instrument (sterilized cotton swab 50) by the rays of laser light 39A and 39B with which irradiation is performed from the pair of laser pointers 38A and 38B.

Further, in the robot system 100 according to the sixth embodiment, the virtual model showing the position information of the medical test instrument and/or the medical examination instrument is displayed on the first display 103 as the fourth video information 103D. This makes it possible for the operator to easily understand the distance between the patient, and the distal end portion of the medical test instrument and/or the medical examination instrument (sterilized cotton swab 50).

At this time, the controller 110 displays the virtual patient 60, which is a virtual model of the medical target site of the patient, on the first display 103, thereby it is possible to more easily understand the distance between the patient, and the distal end portion of the medical test instrument and/or the medical examination instrument (sterilized cotton swab 50).

### [First Modification Example]

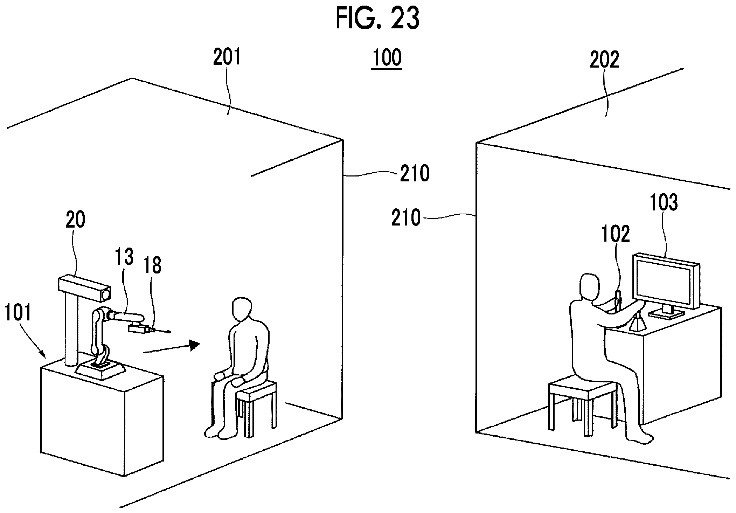

Next, a first modification example of the robot system according to the sixth embodiment will be described below with reference to FIG. 23.

FIG. 23 is a schematic diagram illustrating a schematic configuration of a robot system according to the first modification example in the sixth embodiment.

As illustrated in FIG. 23, the robot system 100 in the first modification example has the same basic configuration as the robot system 100 according to the sixth embodiment, but the first modification example is different from the sixth embodiment in that a robot 101 is configured by a vertical articulated robot.

The robot system 100 in the first modification example, which is configured as described above, also has the similar action and effect to those of the robot system 100 according to the sixth embodiment.

### (Seventh Embodiment)

In a robot system according to a seventh embodiment, a robot further includes a first audio input and a first audio output in the robot system according to the sixth embodiment (including the first modification example). In addition, a second audio input and a second audio output are further disposed in a second space. A controller outputs audio information input to the first audio input, to the second audio output, and outputs audio information input to the second audio input, to the first audio output.

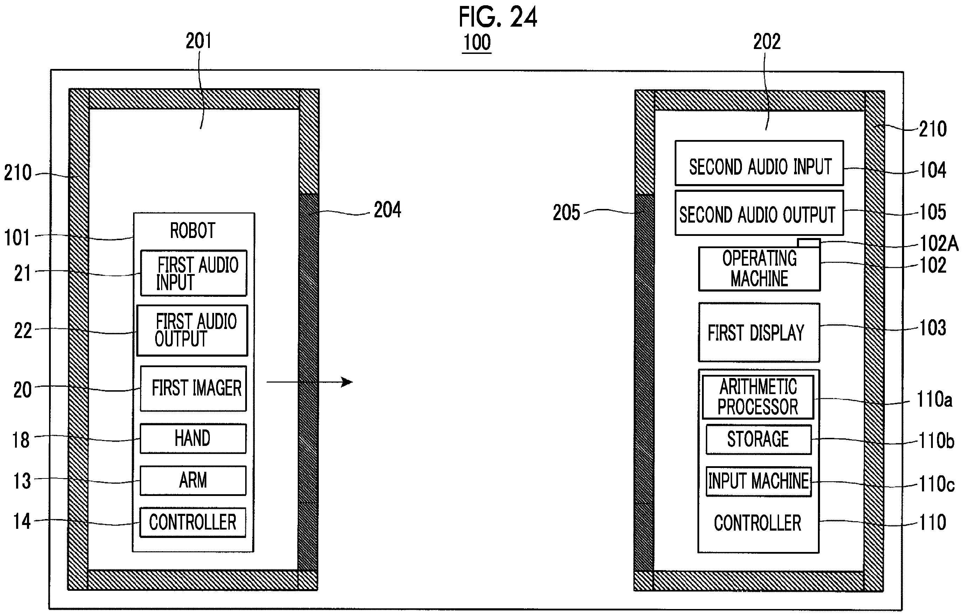

An example of the robot system according to the seventh embodiment will be described below with reference to FIG. 24.

### [Configuration of Robot System]

FIG. 24 is a schematic diagram illustrating a schematic configuration of the robot system according to the seventh embodiment.

As illustrated in FIG. 24, the robot system 100 according to the seventh embodiment has the same basic configuration as the robot system 100 according to the sixth embodiment. The seventh embodiment is different from the sixth embodiment in that the robot 101 includes a first audio input 21 and a first audio output 22, and that a second audio input 104 and a second audio output 105 are disposed in the second space 202.

The first audio input 21 and the second audio input 104 may be configured, for example, by a microphone. In addition, the first audio output 22 and the second audio output 105 may be configured by a speaker.

The second audio input 104 and the second audio output 105 may be configured by headphones (headsets) with a microphone. In addition, when the first display 103 is configured by a head-mounted display, the second audio input 104 and the second audio output 105 may be configured by a microphone and headphones attached to the head-mounted display.

The robot system 100 in the seventh embodiment, which is configured as described above, also has the similar action and effect to those of the robot system 100 according to the sixth embodiment.

Further, in the robot system 100 according to the seventh embodiment, the robot 101 is provided with the first audio input 21 and the first audio output 22, and the second audio input 104 and the second audio output 105 are disposed in the second space 202. Thus, it is possible to perform communication between the patient and the operator.

As a result, for example, when the operator is a medical staff, or when there is a medical staff beside the operator, it is possible to perform medical practice such as inquiries, auscultation, transmission of test results, communication of treatment policies, and the like.

### (Eighth Embodiment)

In a robot system according to an eighth embodiment, a robot further includes a second display in the robot system according to either the sixth embodiment (including the first modification example) or the seventh embodiment. In addition, a second imager is further disposed in the second space, and the controller displays image information and/or video information obtained by picking-up of the second imager, on the second display in (α).

In a control method for a robot system according to the eighth embodiment, a robot further includes a second display in the control method for the robot system according to either the sixth embodiment (including the first modification example) or the seventh embodiment. In addition, a second imager is further disposed in the second space, and, in (α), the second display displays image information and/or video information obtained by picking-up of the second imager.

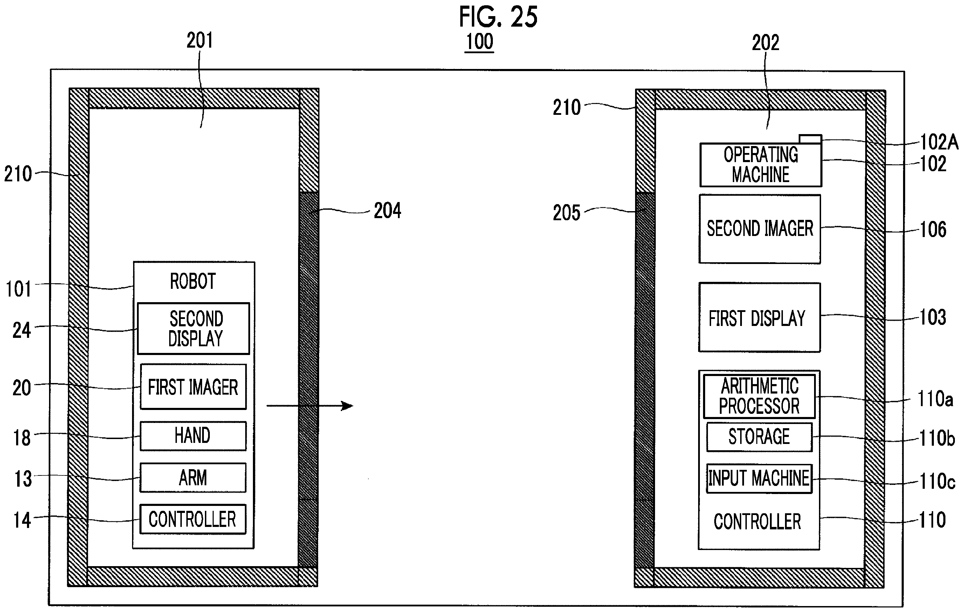

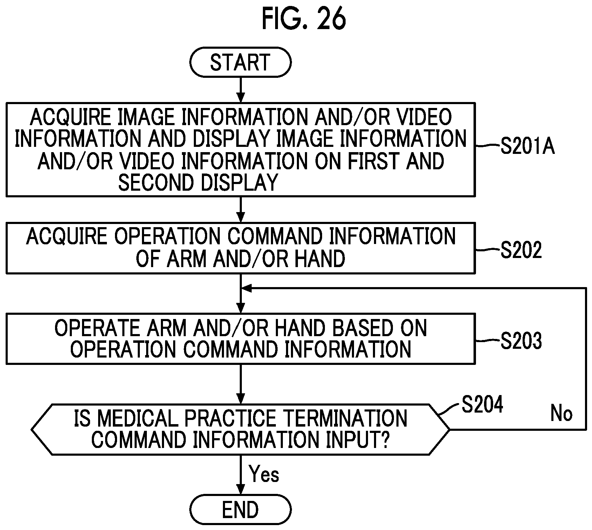

An example of the robot system according to the eighth embodiment will be described below with reference to FIGS. 25 and 26.

### [Configuration of Robot System]

FIG. 25 is a schematic diagram illustrating a schematic configuration of the robot system according to the eighth embodiment.

As illustrated in FIG. 25, a robot system 100 according to the eighth embodiment has the same basic configuration as that of the robot system 100 according to the sixth embodiment. The eighth embodiment is different from the sixth embodiment in that a robot 101 further includes a second display 24, and that a second imager 106 is further disposed in the second space 202.

The second display 24 displays image information and/or video information obtained by picking-up of the second imager 106. For example, a stationary type display may be configured as the second display 24.

The second imager 106 picks up an image and/or a video, and outputs the image information and/or video information obtained by pickup to the second display 24 via the controller 110 and the controller 14. For example, a video camera may be used as the second imager 106.

### [Operations, and Actions and Effects of Robot System]

Next, the operation and the action and effect of the robot system 100 according to the eighth embodiment will be described in detail with reference to FIGS. 25 and 26. The following operations are performed by the arithmetic processor 110a of the controller 110 reading the program stored in the storage 110b.

FIG. 26 is a flowchart illustrating an example of the operation of the robot system according to the eighth embodiment.

As illustrated in FIG. 26, the operation of the robot system 100 according to the eighth embodiment is basically the same as that of the robot system 100 according to the sixth embodiment. The eighth embodiment is different from the sixth embodiment in that the controller 110 executes a process of Step S201A instead of the process of Step S201.

Specifically, the controller 110 acquires the image information and/or the video information obtained by picking-up of the first imager 20, and displays the image information and/or the video information on the first display 103. In addition, the controller 110 acquires the image information and/or the video information obtained by picking-up of the second imager 106, and displays the image information and/or the video information on the second display 24 (Step S201A).

The robot system 100 in the eighth embodiment, which is configured as described above, also has the similar action and effect to those of the robot system 100 according to the sixth embodiment.

Further, in the robot system 100 according to the eighth embodiment, the robot 101 further includes the second display 24, and the second imager 106 is further disposed in the second space 202.

This makes it possible to communicate between the patient and the operator (medical staff).

From the above description, many improvements or other embodiments of the present disclosure will be apparent to those skilled in the art. Accordingly, the above description should be construed as only an example and is provided for the purpose of teaching, to those skilled in the art, the best forms of carrying out the present disclosure. The details of the structures and/or functions can be substantially changed without departing from the present disclosure.

### (Ninth Embodiment)

Next, a robot system 400 according to a ninth embodiment will be described.

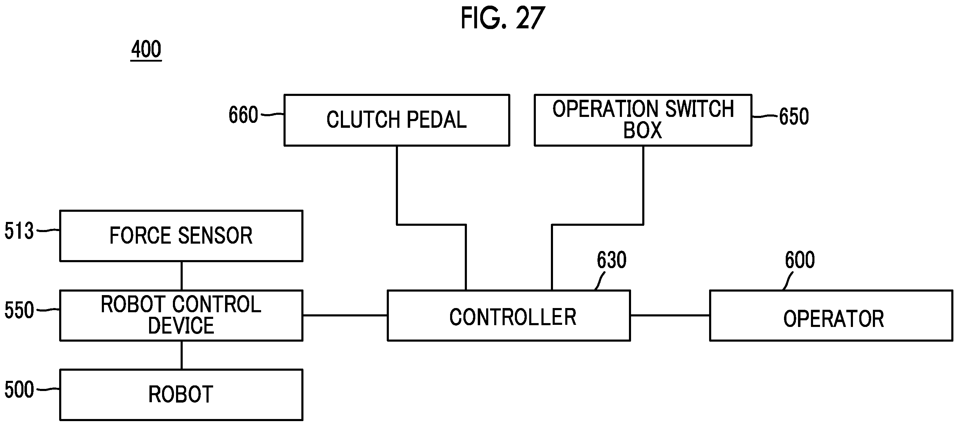

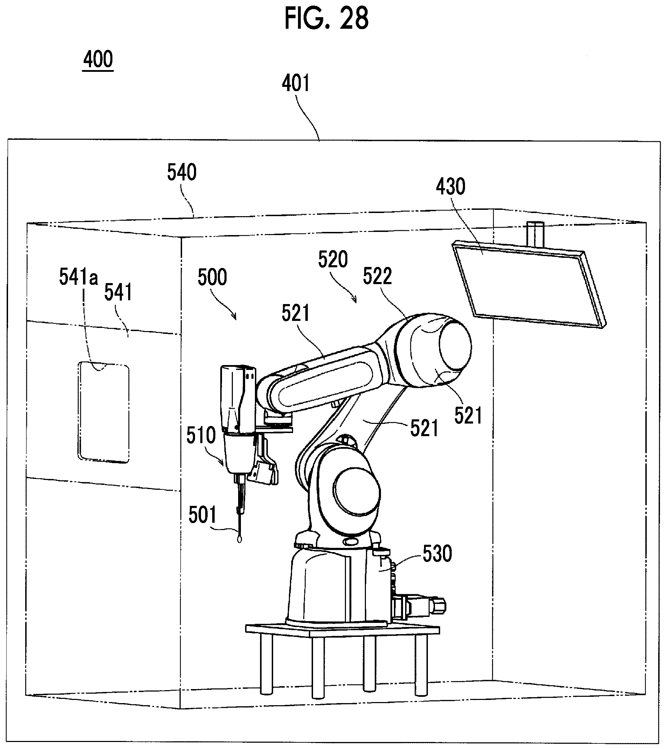

As illustrated in FIGS. 27 and 28, the robot system 400 includes a robot 500. As illustrated in FIG. 28, the robot 500 is disposed in a first space 401. The robot 500 includes an arm 520 with a hand 510 that holds a medical test instrument 501. Further, a second display 430 is disposed in the first space 401.

The arm 520 includes a plurality of link portions 521. The plurality of link portions 521 are connected to each other by joints 522. A plurality of joints 522 are provided. Further, the robot 500 is configured by a vertical articulated robot. Further, each of the plurality of joints 522 is provided with a motor (not illustrated). Further, the arm 520 is mounted on a base 530. The robot 500 may be configured by a dual-arm robot, a horizontal articulated robot, or the like.

Further, the hand 510 is configured by, for example, a chuck for holding (gripping) the test instrument 501.

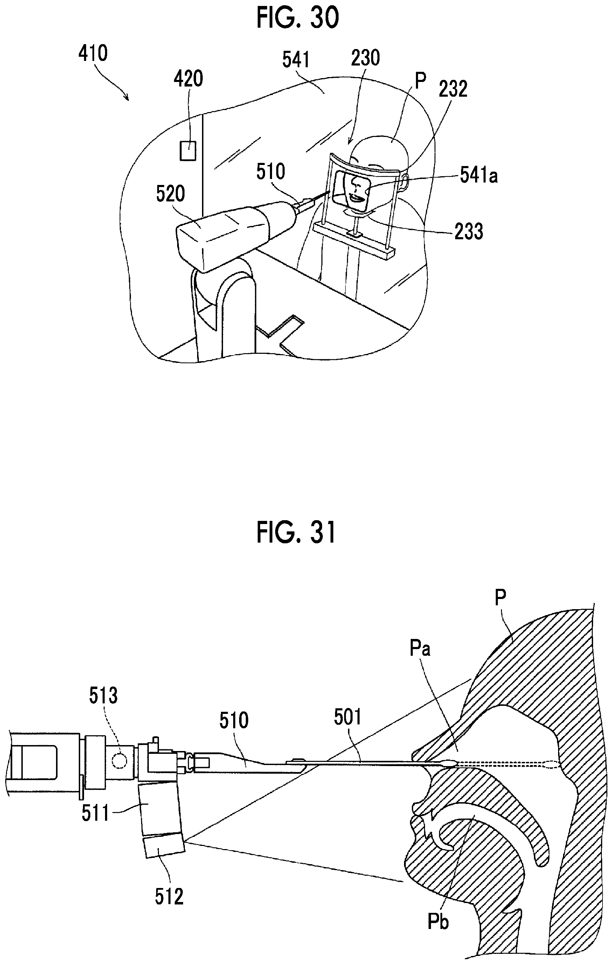

Further, the test instrument 501 includes a test instrument 501 that collects a specimen from at least one of a nasal cavity Pa and an oral cavity Pb of a patient P (see FIG. 31). In the ninth embodiment, the test instrument 501 is a sterilized cotton swab (swab) for collecting a specimen from the nasal cavity Pa of the patient P.

Further, the robot 500 is disposed in a system housing 540.

Further, a shielding plate 541 is disposed between the robot 500 and the patient P. The shielding plate 541 is installed in the system housing 540 that surrounds the robot 500. Further, the shielding plate 541 is made of a transparent member such as a glass plate, and is provided with an opening 541a.

The position and size of the opening 541a are set such that the opening 541a is located in at least one of the nasal cavity Pa and the oral cavity Pb of the patient P (the nasal cavity Pa in the ninth embodiment).

Further, a positioning device 230 (see FIG. 30), which is not illustrated in FIG. 28, is installed between the shielding plate 541 and the patient P. The positioning device 230 includes an abutting target portion 232 and a chin rest 233.

In the positioning device 230, the patient P abuts the forehead on the abutting target portion 232 and puts the chin on the chin rest 233, so that at least one of the nasal cavity Pa and the oral cavity Pb of the patient P (the nasal cavity Pa in the ninth embodiment) is positioned within a range (opening 541a) set in advance. This makes it possible to easily perform positioning of at least one of the nasal cavity Pa and the oral cavity Pb of the patient P (the nasal cavity Pa in the ninth embodiment).

Further, as illustrated in FIG. 27, a robot control unit 550 that controls the robot 500 is provided. A command is input to the robot control unit 550 from an operator 600, which will be described later. The robot 500 operates based on a command input from the operator 600.

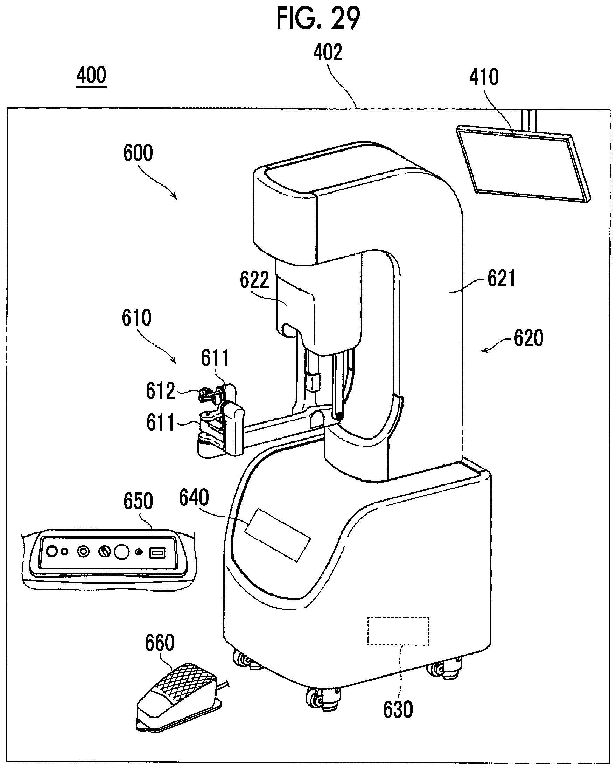

Further, as illustrated in FIG. 29, the robot system 400 includes the operator 600. The operator 600 receives an operation on the test instrument 501 in a second space 402 different from the first space 401. The operator 600 includes an operation handle 610 and a clutch pedal 660. The operation handle 610 constitutes an operation handle for an operator (such as a doctor) to input a command.

The operation handle 610 operates the robot 500. Further, the operation handle 610 receives an operation amount on the robot 500. For example, one operation handle 610 is provided.

Further, the operation handle 610 includes a plurality of link portions 611. When any one of the plurality of link portions 611 is operated (rotated), any one of the plurality of joints 522 of the arm 520 rotates. That is, the operator 600 and the robot 500 are configured by a master-slave method.

Further, the operator 600 includes a gantry 620. The gantry 620 includes a support column 621 configured to bend upward toward the operator (approximately in an L-shape). An arm portion 622 having a substantially L-shape is provided below the bent portion of the support column 621. The operation handle 610 is attached to the arm portion 622.

Further, a controller 630 is provided in the gantry 620. Further, a power supply unit (not illustrated) and the like are provided in the gantry 620.

Further, an operation panel 640 is provided on the outer surface of the gantry 620. By operating the operation panel 640, basic operation instructions, control switching, setting, and the like of the robot 500 and the operator 600 are performed.

Further, an operation switch box 650 is provided in the vicinity of the operator. By operating the operation switch box 650, operations such as zooming of the first imager 511, which will be described later, are performed.

Further, the clutch pedal 660 is provided in the vicinity of the operator. A clutch operation of not operating the robot 500 and only operating the operator 600 is performed in a manner that the clutch pedal 660 is depressed while the operator holds the operation handle 610 (link portion 612 operated by an operator such as a doctor with a finger among the plurality of link portions 611).

Here, in the ninth embodiment, the robot system 400 includes the controller 630. The controller 630 operates the test instrument 501 by controlling the robot 500 in response to the operation received by the operator 600. Specifically, as illustrated in FIGS. 30 and 31, the robot 500 is driven in accordance with the operation received by the operation of the operation handle 610 by the operator. In detail, the test instrument 501 held by the hand 510 is inserted into the nasal cavity Pa of the patient P, and the test instrument 501 comes into contact with the back side of the nasal cavity Pa (see the test instrument 501 indicated by a dotted line in FIG. 31). Then, a specimen is collected by rotating the test instrument 501 together with the hand 510. Then, the test instrument 501 is removed from the nasal cavity Pa of the patient by moving the hand 510 to be spaced from the patient P.

Further, in the ninth embodiment, as illustrated in FIG. 31, a first imager 511 is provided. The first imager 511 is provided at the hand 510 or the arm 520 (the hand 510 in the ninth embodiment), and picks up an image of at least one of the nasal cavity Pa and the oral cavity Pb (the nasal cavity Pa in the ninth embodiment) . The first imager 511 is configured by, for example, a camera that picks up a two-dimensional image. The first imager 511 may be configured by a camera that picks up a three-dimensional image. In addition, the first imager 511 picks up an image of the entire face of the patient P including the nasal cavity Pa. Further, the first imager 511 picks up an image of the test instrument 501 gripped by the hand 510. Further, the first imager 511 is disposed below the hand 510.

Further, in the ninth embodiment, as illustrated in FIG. 29, a first display 410 is disposed in the second space 402. The first display 410 includes, for example, a liquid crystal monitor or the like. Further, the first display 410 is disposed in the vicinity of the operator 600. This makes it possible for an operator such as a doctor to visually recognize the first display 410 while operating the operator 600.

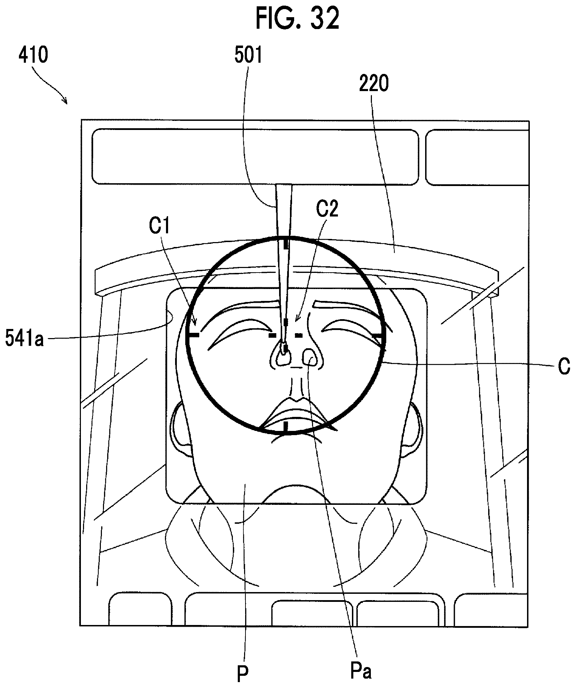

Then, in the ninth embodiment, as illustrated in FIG. 32, the controller 630 displays a pickup image obtained by picking-up of the first imager 511, on the first display 410. That is, the nasal cavity Pa of the patient P picked up by the first imager 511 and the test instrument 501 gripped by the hand 510 appear on the first display 410.

Further, in the ninth embodiment, as illustrated in FIG. 31, an irradiator 512 is provided at the hand 510. The irradiator 512 performs irradiation with light at a position at which the test instrument 501 is inserted into the patient P (the facial portion including the nasal cavity Pa in the ninth embodiment). The irradiator 512 is provided below, for example, the first imager 511 provided at the hand 510. Further, the irradiator 512 is made of, for example, an LED. Then, the first imager 511 picks up an image of the face of the patient P in a state of being irradiated with light.

As a result, in the ninth embodiment, the controller 630 displays, on the first display 410, the image of the patient P irradiated with the light, the image obtained by picking-up of the first imager 511. The irradiator 512 performs irradiation with light during a period in which the test instrument 501 collects a specimen of the patient P.

Further, in the ninth embodiment, the hand 510 is provided with a force sensor 513 (force sense sensor) that detects the force from the test instrument 501 abutting on the patient P. Then, as illustrated in FIG. 32, the controller 630 displays the detection result of the force sensor 513 on the first display 410. Further, the controller 630 displays an insertion depth of the test instrument 501 into the patient P, on the first display 410.

Specifically, in the ninth embodiment, the controller 630 displays, on the first display 410, a circle C indicating the detection result of the force sensor 513 and the insertion depth of the test instrument 501 into the patient P. Then, the controller 630 changes the color of the circle C in accordance with the magnitude of the force detected by the force sensor 513. Further, the controller 630 changes the diameter of the circle C in accordance with the insertion depth of the test instrument 501. A line C1 toward the center of the circle C is added to the circle C at intervals of 90 degrees. Further, a marker C2 (four lines) indicating the center is disposed at the center of the circle C.

Specifically, the controller 630 changes the color of the circle C in order of blue, yellow, and red as the force detected by the force sensor 513 increases. The color is changed continuously (gradually), for example. Further, the controller 630 gradually decreases the radius of the circle C as the distal end of the test instrument 501 gripped by the hand 510 approaches the patient P. After the distal end of the test instrument 501 gripped by the hand 510 has reached the vicinity (predetermined distance) of the patient P, the controller 630 starts control to gradually reduce the radius of the circle C. Further, the controller 630 rotates the circle C to correspond to the rotation of the hand 510.

Further, in the ninth embodiment, as illustrated in FIG. 30, a profile imager 420 that picks up an image of the profile of the patient P is disposed in the first space 401. The profile imager 420 is disposed, for example, in the vicinity of the shielding plate 541 and on the patient P side of the shielding plate 541. Further, the profile imager 420 includes, for example, a 2D camera.

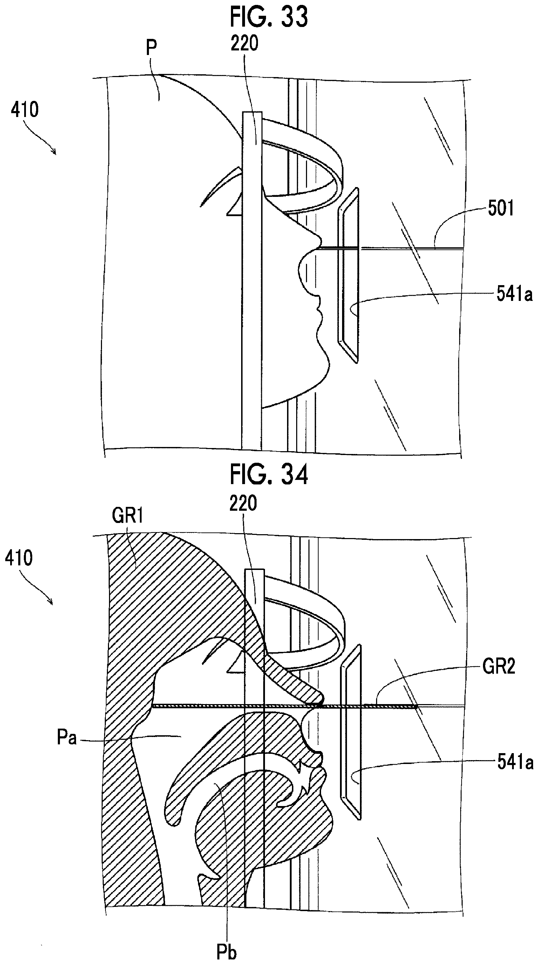

Then, as illustrated in FIG. 34, in the ninth embodiment, the controller 630 displays a model image GR1 and a model image GR2 on the first display 410 by superimposing the model image GR1 and the model image GR2 on an image of the profile (see FIG. 33) of the patient P, which has been picked up by the profile imager 420. The model image GR1 virtually represents the cross section of the profile of the patient P, and the model image GR2 virtually represents the test instrument 501 inserted into the patient P. The image (model image GR1 and model image GR2) in FIG. 34 and the image (circle C) in FIG. 32 are displayed to be adjacent to each other on the first display 410.

Specifically, the contour of the profile is acquired by performing image processing on the image of the profile of the patient P, which has been picked up by the profile imager 420. Then, the model image GR1 that virtually represents the cross section of the profile and is stored in a storage unit or the like in advance is superimposed on the image of the profile of the patient P. The model image GR1 is configured to be translucent, for example, and it is possible to visually recognize the profile of the patient P through the model image GR1. Further, the model image GR1 shows a cross section of the nasal cavity Pa and the oral cavity Pb.

Further, the position (posture, position of the distal end, and the like) of the test instrument 501 is acquired from the robot coordinates of the robot 500. Then, the model image GR2 that virtually represents the test instrument 501 is superimposed on the image of the profile of the patient P based on the acquired position. This makes it possible to visually recognize the position of the test instrument 501 in the nasal cavity Pa in the model image GR1. Further, the model image GR2 is configured to be translucent, for example.

The circle C indicating the detection result of the force sensor 513 and the insertion depth of the test instrument 501 into the patient P, and the model image GR1 and the model image GR2 can be set to be displayed or hidden by the operation panel 640, the operation switch box 650, or the like.

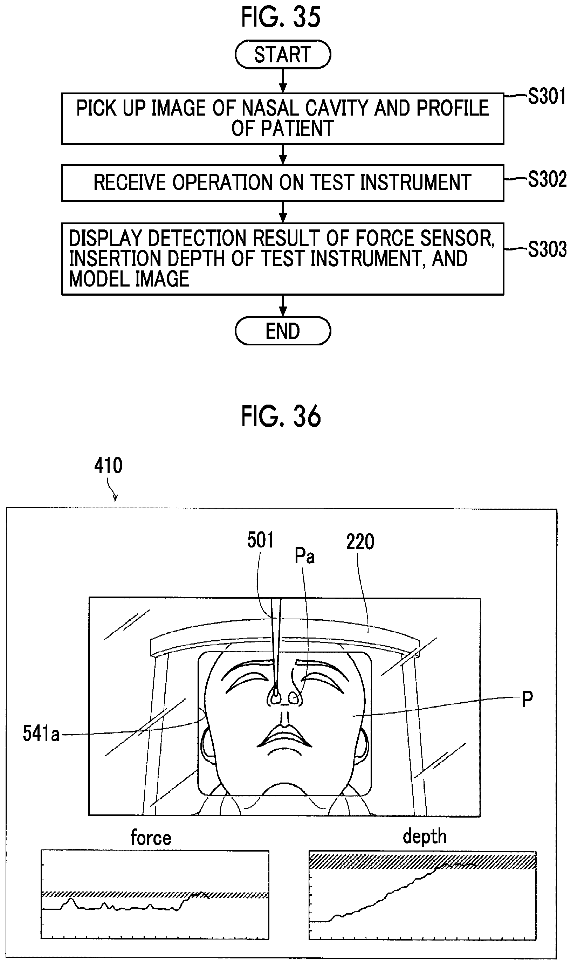

Next, a control method for the robot system 400 will be described with reference to FIG. 35. It is assumed that the patient P is located in advance in front of the robot 500 (shielding plate 541) in the first space 401.

First, in Step S301, the first imager 511 provided at the hand 510 or the arm 520 (the arm 520 in the ninth embodiment) picks up an image of at least one (the nasal cavity Pa in the ninth embodiment) of the nasal cavity Pa and the oral cavity Pb of the patient P. As a result, the image of the nasal cavity Pa of the patient P is displayed on the first display 410 disposed in the second space 402. At this time, the irradiator 512 provided at the hand 510 irradiates the nasal cavity Pa of the patient P with light.

Further, the profile imager 420 disposed in the first space 401 picks up an image of the profile of the patient P. Then, the profile of the patient P is displayed on the first display 410.

Next, in Step S302, an operation on the test instrument 501 is received in the second space 402 different from the first space 401. The operator operates the test instrument 501 while checking the nasal cavity Pa of the patient P displayed on the first display 410.

Next, in Step S303, the circle C indicating the detection result of the force sensor 513 and the insertion depth of the test instrument 501 into the patient P is displayed on the first display 410. Further, the model image GR1 that virtually represents the cross section of the profile of the patient P and the model image GR2 that virtually represents the test instrument 501 inserted in the patient P are displayed on the first display 410 with being superimposed on the image of the profile of the patient P, which has been picked up by the profile imager 420.

Then, the test instrument 501 is operated by controlling the robot 500 in response to the received operation. Then, the model image GR2 that virtually represents the test instrument 501 inserted into the patient P is moved on the first display 410 so as to correspond to the movement of the test instrument 501. Further, the color and the radius of the circle C are changed in accordance with the detection result of the force sensor 513 and the insertion depth of the test instrument 501 into the patient P.

The operations of Steps S301 to S303 are repeated until the collection of the specimen from the patient P is terminated.

### [Effect of Ninth Embodiment]

In the ninth embodiment, it is possible to obtain effects as follows.

In the ninth embodiment, as described above, the medical staff or the like can operate the robot 500 by the operator 600 in the second space 402 isolated from the patient P. Thus, it is possible to suppress the occurrence of a situation in which the medical staff or the like comes into contact with a patient P suspected of being infected with a virus or the like. Therefore, it is possible to sufficiently suppress the infection of a medical staff and the like in a hospital.

Further, in the ninth embodiment, as described above, the controller 630 displays a pickup image obtained by picking-up of the first imager 511, on the first display 410. As a result, the operator who operates the robot 500 can operate the robot 500 while visually recognizing the pickup image obtained by picking-up of the first imager 511. As a result, it is possible to appropriately operate the robot 500.

Further, in the ninth embodiment, as described above, the controller 630 displays the detection result of the force sensor 513 on the first display 410. As a result, the operator who operates the robot 500 can operate the robot 500 while visually recognizing the detection result of the force sensor 513 displayed on the first display 410. As a result, it is possible to operate the robot 500 so as not to apply an excessive force to the patient P.

Further, in the ninth embodiment, as described above, the controller 630 displays the insertion depth of the test instrument 501 into the patient P, on the first display 410. Thus, the operator who operates the robot 500 can operate the robot 500 while visually recognizing the insertion depth of the test instrument 501, which has been displayed on the first display 410. As a result, it is possible to move the test instrument 501 to an appropriate position in the nasal cavity Pa of the patient P.

Further, in the ninth embodiment, as described above, the controller 630 displays, on the first display 410, the circle C indicating the detection result of the force sensor 513 and the insertion depth of the test instrument 501 into the patient P, changes the color of the circle C in accordance with the magnitude of the force detected by the force sensor 513, and changes the diameter of the circle C in accordance with the insertion depth of the test instrument 501. As a result, the detection result of the force sensor 513 and the insertion depth of the test instrument 501 are displayed by the common circle C. Thus, it is possible to suppress the movement of the line of sight of the operator as compared with a case where the detection result of the force sensor 513 and the insertion depth of the test instrument 501 are displayed by separate markers. Therefore, it is possible to improve the visibility when the detection result of the force sensor 513 and the insertion depth of the test instrument 501 are visually recognized.

In addition, in the ninth embodiment, as described above, the controller 630 displays the model image GR1 that virtually represents the cross section of the profile of the patient P and the model image GR2 that virtually represents the test instrument 501 inserted into the patient P, on the first display 410 by superimposing the model image GR1 and the model image GR2 on an image of the profile of the patient P, which has been picked up by the profile imager. Thus, the operator can intuitively understand the insertion depth of the test instrument 501 by visually recognizing the model image GR1 and the model image GR2.

Further, in the ninth embodiment, as described above, the hand 510 is provided with the irradiator 512 that performs irradiation with light at the position at which the test instrument 501 is inserted into the patient P. As a result, before the test instrument 501 is inserted into the patient P, it is possible to easily check the state of the patient P at the position (nasal cavity Pa) at which the test instrument 501 is inserted.

Further, in the ninth embodiment, as described above, the controller 630 displays, on the first display 410, the image of the patient P irradiated with the light, which has been obtained by picking-up of the first imager 511. As a result, even when the operator is located in the second space 402 which is separate from the first space 401 in which the patient P is located, it is possible to easily check the state of the patient P at the position (nasal cavity Pa) at which the test instrument 501 is inserted, by visually recognizing the image of the patient P displayed on the first display 410.

### [Modification Examples]

It should be noted that the embodiments disclosed this time are exemplary in all respects and are not considered to be restrictive. The scope of the present disclosure is shown by the scope of claims rather than the description of the embodiment described above, and further includes all changes (modification examples) within the meaning and scope equivalent to the scope of claims.

For example, in the ninth embodiment, the example in which the test instrument 501 is a test instrument that collects a specimen from at least one (the nasal cavity Pa in the ninth embodiment) of the nasal cavity Pa and the oral cavity Pb of the patient P has been described. The present disclosure is not limited to this. The test instrument 501 may be a test instrument other than the test instrument that collects a specimen from at least one (the nasal cavity Pa in the ninth embodiment) of the nasal cavity Pa and the oral cavity Pb of the patient P, or an examination instrument.

Further, in the ninth embodiment, the example in which the first imager 511 is provided at the hand 510 has been described, but the present disclosure is not limited to this. For example, the first imager 511 may be provided at the arm 520.

Further, in the ninth embodiment, the example in which all the circle C representing the detection result of the force sensor 513 and the insertion depth of the test instrument 501, and the model image GR1 of the cross section of the profile of the patient P and the model image GR2 of the test instrument 501 are displayed on the first display 410 has been described. The present disclosure is not limited to this. For example, only the circle C representing the detection result of the force sensor 513 and the insertion depth of the test instrument 501 may be displayed on the first display 410. Further, only the model image GR1 of the cross section of the profile of the patient P and the model image GR2 of the test instrument 501 may be displayed on the first display 410.

Further, in the ninth embodiment, the example in which both the detection result of the force sensor 513 and the insertion depth of the test instrument 501 are displayed by the circle C displayed on the first display 410 has been described. The present disclosure is not limited to this. For example, the circle C displayed on the first display 410 may be configured to represent only one of the detection result of the force sensor 513 and the insertion depth of the test instrument 501.

Further, in the ninth embodiment, the example in which the detection result of the force sensor 513 and the insertion depth of the test instrument 501 are displayed by the circle C has been described, but the present disclosure is not limited to this. For example, as described in a first modification example of the ninth embodiment illustrated in FIG. 36, the detection result of the force sensor 513 and the insertion depth of the test instrument 501 may be displayed by line graphs (waveforms). In FIG. 36, the horizontal axis is time, and the vertical axis is the magnitude "force" of the force detected by the force sensor 513 (insertion depth "depth" of the test instrument 501). In FIG. 36, the image of the patient P is displayed on the first display 410 together with the line graphs (waveforms).

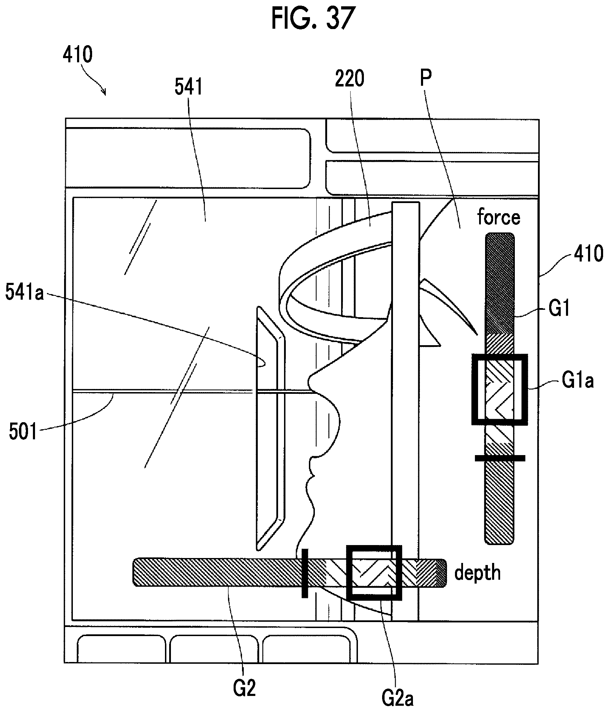

Further, as described in a second modification example of the ninth embodiment illustrated in FIG. 37, the detection result of the force sensor 513 and the insertion depth of the test instrument 501 may be displayed by color-coded gauges G1 and G2, respectively. In the gauge G1 representing the detection result of the force sensor 513, a current value bar (square frame) G1a moves in order of blue, yellow, and red as the magnitude of the force detected by the force sensor 513 increases. In the gauge G2 indicating the insertion depth of the test instrument 501, a current value bar (square frame) G2a moves in order of blue, yellow, and red as the insertion depth of the test instrument 501 increases. In FIG. 37, the colors of blue, yellow, and red are represented by hatching.

Further, in the ninth embodiment, the example in which the irradiator 512 is provided below the hand 510 has been described, but the present disclosure is not limited to this. For example, the irradiator 512 may be provided above or on the side of the hand 510.

Further, in the ninth embodiment, the example in which the controller 630 of the operator 600 performs control of displaying the detection result of the force sensor 513, the insertion depth of the test instrument 501, the model image GR1, and the model image GR2. The present disclosure is not limited to this. A controller other than the controller 630 of the operator 600 may perform control of displaying the detection result of the force sensor 513, the insertion depth of the test instrument 501, the model image GR1, and the model image GR2.

### Industrial Applicability

According to the robot system and the control method for the robot system in the present disclosure, it is possible to sufficiently reduce the infection of a medical staff and the like in a hospital, which is useful in the field of robots.

### Reference Signs List

2A first mounting portion
2B second mounting portion
5a first link
5b second link
12 carriage
13 arm
13A first arm
13B second arm
14 controller
15A first arm portion
15B second arm portion
16 base shaft
17A first wrist portion
17B second wrist portion
18 hand
18A first hand
18B second hand
19 wheels
20 first imager
21 first audio input
22 first audio output
23 container
24 Second display
31 main body
32 Intermediate member
33 Holding member
34 actuator
35 support member
36 camera
37 chuck mechanism
38A laser pointer
38B laser pointer
39B laser light
39A laser light
50 sterilized cotton swab
50A virtual sterilized cotton swab
50B first region
60 virtual patient
100 robot system
101 Robot
102 operating machine
102A release button
103 First display
103A first video information
103B second video information
103C third video information
103D fourth video information
104 Second audio input
105 Second audio output
106 Second imager
110 Controller
110a arithmetic processor
110b storage
110c input machine
201 First space
202 Second space
203 Third space
204 shutter
205 Shutter
206 shutter
207 Protective cover
210 partition wall member
220 base
221 Shielding plate
222 opening
230 Positioning device
231 main body
232 abutting target portion
233 chin rest
300 disinfector
400 robot system
401 First space
402 Second space
410 first display
420 Profile imager
500 robot
501 test instrument
510 hand
511 First imager
512 irradiator
513 Force sensor
520 arm
600 operator
630 controller
C circle
GR1, GR2 model image
J1 rotating joint
J2 rotating joint
J3 linear motion joint
J4 rotating joint
J5 rotating joint
J6 rotating joint
L1 rotation axis
L2 rotation axis
L3 rotation axis
L4 rotation axis
L5 rotation axis
P patient
Pa nasal cavity
Pb oral cavity

## Claims

1. A robot system comprising:
a robot that is disposed in a first space and includes an arm with a hand that holds a medical test instrument;
an operator that receives an operation on the test instrument in a second space different from the first space; and
a controller that operates the test instrument by controlling the robot in response to the received operation.

2. The robot system according to claim 1, wherein
the test instrument includes a test instrument that collects a specimen from at least one of a nasal cavity and an oral cavity of a patient,
the robot system further comprises:
a first imager that is provided at the hand or the arm and picks up an image of at least one of the nasal cavity and the oral cavity; and
a first display that is disposed in the second space, and
the controller displays a pickup image picked up by the first imager, on the first display.

3. The robot system according to claim 1 or 2, further comprising:
a force sensor that is provided at the hand and detects a force from the test instrument abutting on a patient; and
a first display that is disposed in the second space, wherein
the controller displays a detection result of the force sensor on the first display.

4. The robot system according to any one of claims 1 to 3, further comprising:
a first display that is disposed in the second space, wherein
the controller displays an insertion depth of the test instrument into the patient, on the first display.

5. The robot system according to claim 3 or 4, further comprising:
a force sensor that is provided at the hand and detects a force from the test instrument abutting on a patient, wherein
the controller
displays a circle indicating a detection result of the force sensor and an insertion depth of the test instrument into the patient, on the first display,
changes a color of the circle in accordance with magnitude of the force detected by the force sensor, and
changes a diameter of the circle in accordance with the insertion depth.

6. The robot system according to any one of claims 1 to 5, further comprising:
a profile imager that is disposed in the first space and picks up an image of a profile of a patient, wherein
the controller superimposes a model image on the image of the profile of the patient picked up by the profile imager, and displays a result of the superimposition on the first display, the model image virtually representing a cross section of the profile of the patient and the test instrument inserted into the patient.

7. The robot system according to any one of claims 1 to 6, further comprising:
an irradiator that is provided at the hand and performs irradiation with light at a position at which the test instrument is inserted into a patient.

8. The robot system according to claim 7, wherein
the irradiator includes a pair of laser light indicators, and
rays of light with which irradiation is respectively performed from the pair of laser light indicators intersect with each other.

9. The robot system according to claim 7 or 8, further comprising:
a first imager that is provided at the hand or the arm and picks up an image of the patient; and
a first display that is disposed in the second space, wherein
the controller displays the image of the patient irradiated with the light, on the first display, the image picked up by the first imager.

10. The robot system according to any one of claims 1 to 9, wherein
the robot is disinfected in a third space different from the first space and the second space.

11. The robot system according to claim 10, further comprising:
a disinfector that is provided in the third space and disinfects the robot.

12. The robot system according to claim 10 or 11, wherein
the robot is disinfected by itself.

13. The robot system according to any one of claims 1 to 12, wherein
the robot is covered with a protective cover.

14. The robot system according to any one of claims 1 to 13, wherein
the operator receives an operation of holding the test instrument by the hand and releasing the holding.

15. The robot system according to any one of claims 1 to 14, further comprising:
a carriage on which the arm is mounted.

16. The robot system according to any one of claims 1 to 15, further comprising:
a first audio input and a first audio output provided in at least one of the robot and the first space; and
a second audio input and a second audio output provided in the second space, wherein
the controller
causes the second audio output to output audio information input to the first audio input, and
causes the first audio output to output audio information input to the second audio input.

17. The robot system according to any one of claims 1 to 16, further comprising:
a second imager provided in the second space; and
a second display that is provided in at least one of the robot and the first space and displays an image picked up by the second imager.

18. The robot system according to any one of claims 1 to 17, further comprising:
a container that is provided in the robot and contains at least one of a drug, a food, a reagent, a specimen, and the test instrument.

19. The robot system according to any one of claims 1 to 18, wherein
the operator and the robot are configured by a master-slave method.

20. A control method for a robot system including an arm that is disposed in a first space and includes a hand that holds a medical test instrument, the control method comprising:
receiving an operation on the test instrument in a second space different from the first space; and
operating the test instrument by controlling the robot in response to the received operation.

21. A robot system comprising:
a robot that is disposed in a first space and includes an arm with a hand that holds a medical test instrument;
an imager that is provided at the hand or the arm and picks up an image of at least one of a nasal cavity and an oral cavity;
an operator that receives an operation on the test instrument in a second space different from the first space;
a controller that operates the test instrument by controlling the robot in response to the received operation; and
a display disposed in the second space, wherein
the controller displays a pickup image picked up by the imager, on the display.
